# EUROPEAN PATENT SPECIFICATION

(11) **EP 4 081 225 B1**
(45) Date of publication and mention of the grant of the patent: **01.07.2026**
(21) Application number: 20848840.3
(22) Date of filing: 22.12.2020
(51) Int. Cl.: A61K 31/739, A61P 3/04, A23L 33/135, A23L 33/00, A23K 10/16, A23K 20/158, A23K 20/163, A23L 33/115, A23L 33/12

(54) **USES OF LIPOTEICHOIC ACID FROM BIFIDOBACTERIA**
VERWENDUNGEN VON LIPOTEICHOICSÄURE AUS BIFIDOBAKTERIEN
UTILISATIONS D'ACIDE LIPOTÉICHOÏQUE À PARTIR DE BIFIDOBACTÉRIES

(30) Priority: 27.12.2019 EP 19383202
(43) Date of publication of application: 02.11.2022
(73) Proprietor: Biopolis, S.L., 46980 Paterna (Valencia) (ES)
(72) Inventor: MARTORELL GUEROLA, Patricia, 46980 PATERNA (Valencia) (ES); BALAGUER VIDAL, Ferran, 46980 PATERNA (Valencia) (ES); ENRIQUE LÓPEZ, María, 46980 PATERNA (Valencia) (ES); TORTAJADA SERRA, Marta, 46980 PATERNA (Valencia) (ES); RAMON VIDAL, Daniel, 46980 PATERNA (Valencia) (ES); BARRENA CASTILLO, Marta, 46980 PATERNA (Valencia) (ES); HAMMANN, Blake, 46980 PATERNA (Valencia) (ES); ALVÁREZ PÉREZ, Beatriz, 46980 PATERNA (Valencia) (ES); CHENOLL CUADROS, Empar, 46980 PATERNA (Valencia) (ES)
(74) Representative: Pons IP
(86) International application number: PCT/EP2020/087615
(87) International publication number: WO 2021/130219

(56) References cited:
- EP-A1- 1 260 227
- EP-A1- 3 048 165
- CN-A- 103 911 326
- PATRICIA MARTORELL ET AL: "Probiotic Strain Bifidobacterium animalis subsp. lactis CECT 8145 Reduces Fat Content and Modulates Lipid Metabolism and Antioxidant Response in Caenorhabditis elegans", JOURNAL OF AGRICULTURAL AND FOOD CHEMISTRY, vol. 64, no. 17, 4 May 2016 (2016-05-04), US, pages 3462 - 3472, XP055326251, ISSN: 0021-8561, DOI: 10.1021/acs.jafc.5b05934
- HABU YOSHIKI ET AL: "Structural Studies of Cell Wall Polysaccharides from Bifidobacterium breve YIT 4010 and Related Bifidobacterium Species1", JOURNAL OF BIOCHEMISTRY, vol. 102, no. 6, 1 December 1987 (1987-12-01), GB, pages 1423 - 1432, XP055865753, ISSN: 0021-924X, Retrieved from the Internet <URL:http://dx.doi.org/10.1093/oxfordjournals.jbchem.a122188> DOI: 10.1093/oxfordjournals.jbchem.a122188
- H IWASAKI ET AL: "Structure of macroamphiphiles from several Bifidobacterium strains.", JOURNAL OF BACTERIOLOGY, vol. 172, no. 2, 1 January 1990 (1990-01-01), pages 845 - 852, XP055682593, ISSN: 0021-9193, DOI: 10.1128/JB.172.2.845-852.1990

## Description

The present invention falls within the food, feed and pharmaceutical industries. It particularly relates to a lipoteichoic acid (LTA) from Bifidobacteria which has fat reduction activity, thus being useful for exploitation in the following application areas: food and beverages, animal feed, including pet food, nutritional supplements, infant nutrition, cosmetics (including nutricosmetics), medical foods and pharmaceutical and veterinary applications, among others.

### BACKGROUND ART

According to the World Health Organization, in 2016 13% of the world population, more than 1.9 billion people, were overweight, of these over 650 million adults were obese. Despite improved clinical and epidemiological knowledge of this problem, the prevalence of obesity and overweight has increased significantly in industrialized and developing countries. Obesity and overweight are metabolic and nutritional disorders with serious health consequences, overweight being a degree of obesity. Obesity is a recognized high-risk factor in the incidence of various chronic diseases/disorders such as hypertension, ischemic heart disease, brain stroke, type-2 diabetes and certain forms of cancer, which are important causes of morbidity and mortality in developing countries in the Western world.

In the struggle against overweight and obesity, the food industry has introduced new ingredients in order to help consumers maintain an appropriate weight. In the field of research and new product development, one option has been to add certain ingredients that act by inhibiting the accumulation of energy as fat, either by decreasing fat absorption or formation, or by stimulating fat mobilization with increased lipolysis, or by improving lipid oxidation rates.

Another strategy that acts positively on the prevention or treatment of overweight and obesity is to control and/or reduce appetite by the induction of satiety, activating the metabolic regulation of appetite, or by making harder for the body to absorb fat from the ingested food, modulating the capacity if the intestines to absorb certain types of fat.

Moreover, the intestinal microbiota and probiotics have been shown to have a positive effect on health, namely obesity. Studies indicate that the intestinal microbiota is a factor that plays a role in regulating body weight and obesity-associated diseases/disorders. Lactic acid bacteria (LAB) and also species of the genus *Bifidobacterium* are normal inhabitants of the human adult gastrointestinal tract. Several works (EP1945235, EP2898061, EP2129386, EP3048165) have shown that selected strains from the aforementioned genera generally termed probiotics, can be effective in the modulation and control of human metabolism and obesity although little is known about the precise mechanisms which underlie these biological effects. LTA are macroamphiphilic molecules found as major constituents of bacterial cell walls in Gram-positive bacteria, anchored to cell membranes by its lipidic moieties. The conventional LTA polymer structure is formed by a poly-glycerol phosphate (GroP) backbone, bound to a glycolipid structure or anchor. The polyol phosphate backbone can be substituted with monosaccharides such as glucose or galactose, and amines, such as alanine or glucosamine. Classical LTAs have been classified in major classes (I, II, III, IV) that can be distinguished on the basis of their chemical structure. It is known in the state of the art that LTA I structures are found naturally in different species from phylum *Firmicutes,* including but not limited to *Staphylococcus aureus, Bacillus subtilis* and *Streptococcus* sp. Members of beneficial genera such as *Lactobacillus, Lactococcus* and *Leuconostoc* also show type I LTA structures, with single GroP repeating units. ([Shiraishi et al., 2016, Bioscience of Microbiota, Food and Health, vol 35 (4) 147-161; Scheewind&Missiakas 2014, J. Bacteriology, 196 (6) 1133-1142].

Some microorganisms are known in the state of the art to produce atypical lipoteichoic acids that are generally referred to as lipoglycans, or cell-surface glycolipids. These unconventional LTAs have been grouped as type V LTAs and represent polysaccharides attached to lipids, instead of repeating phosphodiester-linked units. Type V LTAs include macroamphophiles such as lipoglycans, Gro-P-lipoglucogalactofuranan, and succinyl lipomannan. (Schneewind O. and Missiakas D., J Bacteriol, 196 (6), 1133-42 Mar 2014).

Bifidobacterial LTAs are included in type V LTAs group, and have been generally described as lipoglycans formed by gluco-galactan chains bearing monomeric glycerophosphate side chains, joined by a galactosyl glycolipid to the cell membrane. The most likely common structure for the macroamphiphiles has been described as follows: 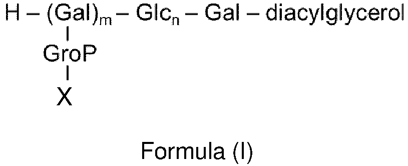 where GroP is glycerophosphate, y m is the number of repeating units of galactofuranan and n is the number of repeating units of glucan (Hiroyoshi Iwasaki, Yoshio Araki, Eiji Ito, Masato Nagaoka and Teruo Yokokura, J. Bacteriology 1990, 845-852).

Several studies have reported the use of LTAs for the treatment and/or prevention of human diseases and/or syndromes. The U.S. patent application US2014323430A1 reports the use of a composition of lipoteichoic acid, preferably from *Bacillus subtilis,* and a bacterial endotoxin for the treatment or prevention of a metabolic disorder and/or a bacterial infection in order to improve milk energy efficiency in ruminant animals. Furthermore, the document refers to a possibility of treating or preventing human metabolic disorders related with abdominal obesity, altered levels of cholesterol and glucose in the blood as well as for the treatment and prevention of bacterial infections.

The U.S. patent application US2012190634A1 reports the use of LTAs from *Lactobacillus* and derived glycolipids as anti-inflammatory agents for the treatment or prevention of inflammatory processes such as diabetes mellitus, due to their function in the inhibition of the production of inflammatory cytokines.

A similar use of LTA from acid lactic bacteria is reported in the U.S. patent application US2004147010A1, namely, the use of LTA for modulating the immune response induced by Gram-negative bacteria allowing the prevention or reduction of inflammatory processes induced by said Gram-negative bacteria.

Finally, the international patent application WO9623896A1 reports the use of an LTA isolated from *Streptococcus* sp. for the treatment of cancer and the treatment or prevention of high blood cholesterol.

The patent application EP1260227 A1 discloses the use of LTA from *B. animalis* for the production of a food product and/or pharmaceutical formulations, which can be used in the treatment and/or prevention of excess weight and obesity and related diseases such as metabolic syndrome, hypertension, glycemia, inflammation, type 2 diabetes, cardiovascular diseases, hypercholesterolemia, hormonal alterations, infertility, etc.

The patent CN103911326 B discloses a *Bacillus coagulans* probiotic preparation obtained by solid fermentation, characterized in that solid state fermentation culture method is: first inoculation *Bacillus coagulans* HM-09 and inoculation of *Bifidobacterium animalis* subspecies *lactis.*

Martorell, P., et al. 2016 (J Agric Food Chem 64: 3462-3472) discloses the functional effect and metabolic targets of a bacterial strain *Bifidobacterium animalis* subsp. *lactis* CECT 8145. This strain significantly reduced total lipids (40.5% ± 2.4) and triglycerides (27.6% ± 0.5). Transcriptomic and metabolomic analyses in nematodes fed with strain CECT 8145 revealed modulation of the energy and lipid metabolism, as well as the tryptophan metabolism (satiety). In conclusion, Martorell, P. et al. describes *B. animalis* subsp. *lactis* strain, CECT 8145, as a promising probiotic for obesity related disorders.

Despite the several efforts made in order to find probiotics that exert beneficial effects on overweight and obesity, there is still a need in the field for finding improved compounds and compositions with fat reduction activity that can be used for the treatment and/or prevention of these conditions and related metabolic disorders.

### DESCRIPTION OF THE INVENTION

The present invention relates to a lipoteichoic acid (LTA) obtained from *Bifidobacterium* and its effect as a fat reduction agent.

The inventors observed that when *Bifidobacterium* is grown in excess of sugars as carbon source, such as glucose, i.e. the glucose is not a limiting nutrient throughout all the phases of the culture, namely lag phase, exponential phase and stationary phase, *Bifidobacterium* acquires the unexpected ability of reducing the fat deposit in the body when it is administered to a subject. As can be seen on Example 1 of the present description, from this moment on the inventors started to analyse the different components of *Bifidobacterium* discovering that in said conditions of excess of glucose, the LTA component of the cell wall suffers, surprisingly, a change in the proportion/ratio of components of LTA which allow the *Bifidobacterium* to acquire said capacity of reducing the fat deposit. Next, the isolation and deep analysis of the LTA structure showed that the LTA coming from *Bifidobacterium* cultured in excess of glucose comprises a molar ratio Alanine/glucose of at least 0.5 and a molar ratio glycerol phosphate/glucose of at least 6.0, ratios which are different in LTAs isolated from *Bifidobacterium* not cultured in excess of glucose (see Example 4 of the present description). Therefore, both *Bifidobacterium* cultured in excess of sugars as carbon source and the LTA isolated thereof show the capacity of reducing the body fat in a subject, and can be used as an active compound in medicaments for use in the treatment and/or prevention of obesity, overweight or related diseases, as well as for the production of cosmetic and food and feed products.

The invention is set out in the appended set of claims.

### LTA of the invention

In view of the foregoing, in one aspect, the present invention relates to lipoteichoic acid (LTA), hereinafter "LTA of the invention", comprising a molar ratio Alanine/glucose of at least 0.5 and a molar ratio glycerol phosphate/glucose of at least 6.0.

In the context of the present invention, the term "lipoteichoic acid" or "LTA" refers to the lipoteichoic acid isolated from a species of the genus *Bifidobacterium* which has been cultured in excess of glucose, i.e. the glucose is not a limiting nutrient throughout all the phases of the culture: lag phase, exponential phase and stationary phase. The glucose monitoring in the culture can be carried out by methods widely known in the state of the art.

As it is known in the state of the art, the LTA isolated from *Bifidobacterium* has the following structure 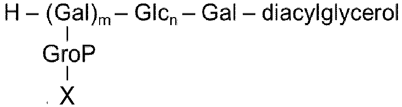 wherein
- GroP is glycerophosphate,
- Gal is galactofuranan,
- each X is independently selected from hydrogen and Alanine (Ala),
- the number of molecules of Alanine is m or m-p, being m the number of repeating units of Gal and p the number of units of Gal in which X is hydrogen,
- Glc is glucan,
- m is the number of repeating units of Gal and is between 10 and 20, preferably, between 11 to 18, and
- n is the number of repeating units of Glc and is between 5 and 20, preferably, between 8 and 12.

The compounds glycerophosphate, Alanine, galactofuranan and glucan are widely known in the state of the art.

As used herein, the term "molar ratio" means the proportions between the different compounds comprising the LTA, i.e., it relates to the number of moles of one substance with the number of moles of another substance. Herein, the LTA of the invention comprises a molar ratio Alanine/glucose of at least 0.5 and a molar ratio glycerol phosphate/glucose of at least 6.0. This means that the number of moles of alanine with respect to the number of moles of glucose in the LTA molecule is at least 0.5, and that the number of moles of glycerol phosphate with respect to the number of moles of glucose in the LTA molecule is at least 6.0. Means for measuring the number of moles of each component in a molecule are widely known in the state of the art and they are routine practice for the skilled person in the art.

In a particular embodiment of the LTA of the invention, the molar ratio Alanine/glucose is at least 0.6, 0.7, 0.8, 0.9 or 1.0.

In a more particular embodiment of the LTA of the invention, the Alanines of the LTA are L-Alanine, D-Alanine or a combination of L- and D- Alanine. The inventors have observed that when the LTA is functional, i.e. it shows the capacity of reducing body fat, most of the Alanines are D-Alanine.

In another particular embodiment of the LTA of the invention, the molar ratio glycerol phosphate/glucose is at least 6.5, 7.0, 7.5, 8.0, 8.5, 9.0, 9.5, 10.0, 10.5, 11.0, 11.5, 12.0 or 12.5.

In a more particular embodiment of the LTA of the invention, the molar ratio glycerol phosphate/glucose is 12.6.

Other structural changes suffered by the LTA isolated from Bifidobacteria cultured in excess of glucose relates to the number of galactoses, glycerol and phosphorous molecules. Thus, in the LTA isolated from Bifidobacteria cultured in excess of glucose, the molar ratio Galactose/glucose is at least 0.8, the mol ratio Glycerol/glucose is at least 1.0, and/or the molar ratio phosphorous/glucose is at least 5.0, 10.0, 15.0, 20.0 or 25.0, preferably the molar ratio phosphorous/glucose is 26.0, more preferably, 26.09.

The LTA of the invention can be obtained from any species of *Bifidobacterium* genus or "Bifidobacteria". The term "Bifidobacteria" as used in the present invention refers to the genus *Bifidobacterium,* a Gram-positive, non-motile, often branched anaerobic bacteria. Thus, the terms *"Bifidobacterium"* and "Bifidobacteria" can be use indistinctly throughout the present description. They are ubiquitous inhabitants of the gastrointestinal tract, vagina and mouth of mammals, including humans. As mentioned previously, some Bifidobacteria are used as probiotics. The term "Bifidobacteria" includes, without limiting to, the following species of bacteria: *B*. *actinocoloniiforme, B. adolescentis, B. angulatum, B. animalis, B. aquikefiri , B. asteroides, B. biavatii , B. bifidum, B. bohemicum, B. bombi, B. boum, B. breve, B. callitrichos, B. catenulatum, B. choerinum, B. commune, B. coryneforme, B. cuniculi, B. crudilactis, B. denticolens, B. dentium, B. eulemuris, B. faecale, B. gallicum, B. gallinarum, B. hapali, B. indicum, B. inopinatum, B. kashiwanohense, B. lemurum, B. longum, B. magnum, B. merycicum, B. minimum, B. mongoliense, B. moukalabense, B. myosotis, B. pseudocatenulatum, B. pseudolongum, B. psychraerophilum, B. pullorum, B. reuteri, B. ruminantium, B. saguini, B. scardovii, B. stellenboschense, B. stercoris , B. saeculare, B. subtile, B. thermacidophilum, B. thermophilum, B. tissieri* and *B. tsurumiense.*

Nevertheless, in a particular embodiment, the LTA is obtained from *Bifidobacterium animalis,* preferably from *Bifidobacterium animalis* subsp. *lactis,* more preferably from the strain *Bifidobacterium animalis* subsp. *lactis* CECT 8145.

The strain *Bifidobacterium animalis* subsp. *lactis* CECT 8145 (also called in the present invention "BPL1" or "BPL0001") was deposited on May 14^{th} 2012 in the Colección Española de Cultivos Tipo (CECT), Parc Cientific Universitat de Valencia, c/ Catedrático Agustín Escardino, 9, 46980 Paterna - Valencia, Spain, according to the conditions of the Budapest Treaty.

Likewise, the LTA is obtained from *Bifidobacterium longum,* preferably, from the strain *Bifidobacterium longum* CECT 7347.

The strain *Bifidobacterium longum* CECT 7347 was isolated from the feces of a healthy breast-fed infant, under three months of age, and deposited by Consejo Superior de Investigaciones Científicas (CSIC) [address: Serrano 142, 28006 Madrid, Spain] on December 20, 2007 according to the conditions of the Budapest Treaty, in the Spanish Type Culture Collection, as International Depositary Authority (Valencia, SPAIN). It was assigned accession number CECT 7347. The scientific classification of the strain of the invention CECT 7347 is: Kingdom: Bacteria Division: Firmicutes; Class: Actinobacteria; Order: Bifidobacteriales, Family: Bifidobacteriaceae, Genus: Bifidobacterium, Species: *Bifidobacterium longum.* The depositor authorizes the applicant (BIOPOLIS S.L.) to refer to the aforementioned deposited biological material in the present patent application and gives his unreserved and irrevocable consent to the deposited material being made available to the public as from the date of filing, or if priority has been claimed, from priority date, of the present patent application.

The LTA of the invention may undergo different treatments, such as heat or lyophilization, in order to sterilize, preserve or extend the shelf life of the LTA. Thus, in a particular embodiment, the LTA of the invention is heat-treated or lyophilized.

The term "heat-treated" as used herein refers to the product that has received thermal processing with continuous or discontinuous heating, by direct or indirect contact with air, fluids, heated surfaces or tanks, including but not limited to, thermal sterilization techniques, such as autoclaving and pasteurization or other industrial treatments including but not limited to extrusion, molding or spray-drying.

The term "lyophilized" as used herein refers to the method of removing free water from the solution containing the LAT-v of the invention by sublimation, subjecting the solution to quick freezing and then removing the ice by light vacuum heating which transforms the ice into steam.

As the skilled person in the art understands, the LTA of the invention can be comprised inside a composition together with other components. Therefore, in another aspect, the present invention relates to a composition comprising the LTA of the invention, hereinafter "composition of the invention".

According to the conventional techniques known to those skilled in the art, the composition according to the present invention may be formulated in combination with an excipient and/or a carrier.

The term "excipient" refers to a substance which helps to absorb any of the components of the composition of the invention, stabilizes said components or helps in the preparation of the composition in the sense of giving it consistency or providing flavours which make them more pleasant. Thus, excipients could have the function of keeping the components bound together, such as for example starches, sugars or celluloses, a sweetening function, a colorant function, the function of protecting the active ingredient, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material which, included in the galenic forms, is added to the active ingredients or to its associations to enable its preparation and stability, modify its organoleptic properties or determine the physical/chemical properties of the composition and its bioavailability. The "excipient" should allow for the activity of the compounds of the composition, that is to say, for it to be compatible with said components. Examples of excipients are agglutinants, fillers, disintegrators, lubricants, coaters, sweeteners, flavorings and colorants. Non-limiting, more specific examples of acceptable excipients are starches, sugars, xylitol, sorbitol, calcium phosphate, steroid fats, talc, silica or glycerine, amongst others.

The term "carrier" is preferably an inert substance. The function of the carrier is to facilitate the incorporation of other compounds, to allow a better dosing and administration or to give consistency and form to the composition. Therefore, the carrier is a substance which is used to dilute any of the components of the composition of the present invention to a determined volume or weight, or even without diluting said components, capable of allowing better dosing and administration or giving consistency and form to the composition.

In another particular embodiment, the composition of the invention is a pharmaceutical composition. In this case, the excipient or the carrier of the composition is a "pharmaceutically acceptable excipient" or a "pharmaceutically acceptable carrier". This means that the carrier or the excipient should allow for the activity of the compounds of the pharmaceutical composition (herein the LTA of the invention), that is to say, for it to be compatible with said components. When the form of presentation is liquid, the pharmaceutically acceptable carrier is the diluent. Likewise, a composition, including its components, is said to be "pharmacologically acceptable" if its administration can be tolerated by a recipient subject, such as a mammal.

The pharmaceutical composition may be administered by any appropriate administration route, to this end, said composition will be formulated in the suitable pharmaceutical form for the selected administration route.

Suitable routes of administration may, for example, include depot, transdermal, oral, rectal, transmucosal, or intestinal administration; parenteral delivery, including intramuscular, subcutaneous, intravenous, intramedullary injections, as well as intrathecal, direct intraventricular, intraperitoneal, intranasal, or intraocular injections. Nevertheless, a preferred route of administration is the oral route.

Pharmaceutical formulations for parenteral administration comprise aqueous solutions of the LTA in water-soluble form. Additionally, the LTA of the invention may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances that increase the viscosity of the suspension, such as sodium carboxymethyl cellulose, sorbitol, or dextran. In some embodiments an active ingredient, such as the LTA of the invention, may be in powder form for constitution with a suitable vehicle, e.g., sterile pyrogen-free (SPF) water, before use. As an illustrative example, for injection, a pharmaceutical composition according to the present invention may be formulated as an aqueous solution, for example in physiologically compatible buffers such as Hanks's solution, Ringer's solution, or physiological saline buffer. For oral administration, a respective pharmaceutical composition can be formulated readily by combining the LTA with pharmaceutically acceptable carriers well known in the art. Such carriers enable the LTA of the invention to be formulated as tablets, pills, lozenges, dragées, capsules, liquids, gels, syrups, slurries, suspensions and the like, for oral ingestion by a patient to be treated.

Pharmaceutical preparations for oral use can be obtained by adding a solid excipient, optionally grinding a resulting mixture, and processing the mixture of granules, after adding suitable auxiliaries, if desired, to obtain tablets or dragée cores. Suitable excipients are, in particular, fillers such as sugars, including lactose, glucose, sucrose, mannitol, or sorbitol; starches and derivatives thereof, such as, corn starch, dextrin and wheat starch, rice starch, potato starch, hydroxypropyl starch, wheat starch, gelatine, gum tragacanth, methyl cellulose, hydroxypropylmethyl-cellulose, sodium carboxymethylcellulose, and/or polyvinylpyrrolidone (PVP); cellulose preparations such as, for example, methylcellulose, carboxylmethylcellulose and hydroxypropylcellulose; inorganic compounds, such as sodium chloride, boric acid, calcium sulfate, calcium phosphate and precipitated calcium carbonate. If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of LTA of the invention doses.

Suitable fluidizing agents include, but are not limited to, magnesium oxide, synthetic aluminium silicate, metasilicic acid, magnesium aluminium oxide, hydrous silicic acid, anhydrous silicic acid, talc, magnesium stearate, and kaolin. Suitable binding agents include, but are not limited to, polyethylene glycol, polyvinyl pyrrolidine, polyvinyl alcohol, gum arabic, tragacanth, sodium alginate, gelatine, and gluten. Suitable stabilisers include, but are not limited to, proteins, such as albumin, protamine, gelatine and globulin; and amino acids and salts thereof. Suitable thickeners include, but are not limited to, sucrose, glycerine, methylcellulose, and carboxymethylcellulose. Suitable pH adjusting agents include, but are not limited to, hydrochloric acid, sodium hydroxide, phosphates, citrates, and carbonates.

Pharmaceutical compositions that can be used orally include, but are not limited to, push-fit capsules made of gelatine, as well as soft, sealed capsules made of gelatine and a plasticiser, such as glycerol or sorbitol. The push-fit capsules may contain the LTA in admixture with filler such as lactose, binders such as starches, and/or lubricants such as talc or magnesium stearate and, optionally, stabilisers. In soft capsules, the LTA may be dissolved or suspended in suitable liquids, such as fatty oils, liquid paraffin, or liquid polyethylene glycols. In addition, stabilisers may be added. All formulations for oral administration should be in dosages suitable for such administration.

For buccal administration, a respective pharmaceutical composition may take the form of tablets or lozenges formulated in conventional manner.

For administration by inhalation, a pharmaceutical composition for use according to the present invention may conveniently be delivered in the form of an aerosol spray presentation from pressurised packs or a nebuliser, with the use of a suitable propellant, e.g., dichlorodifluoromethane, trichlorofluoromethane, dichlorotetrafluoroethane, carbon dioxide or other suitable gas. In the case of a pressurised aerosol the dosage unit may be determined by providing a valve to deliver a metered amount. Capsules and cartridges of e. g. gelatine for use in an inhaler or insufflator may be formulated containing a powder mix of the LTA and a suitable powder base such as lactose or starch.

Alternatively to the pharmaceutical composition, in another particular embodiment the composition of the invention is a nutritional composition, which may comprise a carrier or an excipient as defined above.

In the present invention, the term "nutritional composition" refers to that food, which regardless of providing nutrients to the subject who consumes it, beneficially affects one or more functions of the body, so as to provide better health and wellness. In the present invention, said nutritional composition is intended to ease, reduce, treat and/or prevent obesity, overweight or related diseases, preferably, the related disease is diabetes.

The nutritional composition may be a food. As used herein, the terms "food" and "feed product" are equivalent and can be used indistinctly throughout the present description. The food product can be in liquid, powdery, or solid form. The food may be for human or non-human animal consumption, including infant nutrition, feed and animal food. Foods comprise functional foods commonly known in the art. Examples of foods include, but not limited to, feed, dairy products, vegetable products, meat products, snacks, confectionery products (for example, but not limited to, chocolates, sweets and chewing gum or bubble gum), fodder, drinks, baby food, cereals, fried foods, industrial bakery products and biscuits. Examples of milk products include, but are not limited to, products derived from fermented milk (for example, but not limited to, yogurt or cheese) or non-fermented milk (for example, but not limited to, ice cream, butter, margarine or whey). The vegetable product is, for example, but not limited to, a cereal in any form of presentation, fermented (for example, soy yogurt, oat yogurt, etc.) or unfermented, and a snack. The beverage may be, but is not limited to, non-fermented milk. In a particular embodiment, the food product or food is selected from the group consisting of fruit or vegetable juices, ice cream, infant formula, milk, yogurt, cheese, fermented milk, powdered milk, cereals, baked goods, milk-based products, meat products, confectionery products, animal feed or fodder, and beverages.

The nutritional composition may be a nutritional supplement.

The term "nutritional supplement", synonymous with any of the terms "dietary supplement", "food supplement", "alimentary supplement" or "alimentary complement", refers to products or preparations whose purpose is to supplement the normal diet consisting of sources of concentrated nutrients or other substances with a nutritional or physiological effect. In the present invention, the "substance" which has a nutritional or physiological effect on the individual when the alimentary complement is ingested is the LTA of the invention which is part of the composition of the invention. The nutritional supplement may be in single or combined form and be marketed in dosage form, i.e. in capsules, pills, tablets and other similar forms, sachets of powder, ampoules of liquids and drop dispensing bottles and other similar forms of liquids and powders designed to be taken in a single amount.

There is a wide range of nutrients and other elements that may be present in nutritional supplement including, without limiting to, vitamins, minerals, amino acids, essential fatty acids, fibre, enzymes, plants and plant extracts. Since their role is to complement the supply of nutrients in a diet, they should not be used as a substitute for a balanced diet and intake should not exceed the daily dose expressly recommended by the doctor or nutritionist. The nutritional composition can also be part of the so-called "food for special groups", i.e. foods that meet specific nutritional needs.

The composition of the invention, either the pharmaceutical or the nutritional composition, may further comprise at least a bioactive compound, preferably, a bioactive compound useful in the treatment and/or prevention of obesity, overweight or related diseases, preferably, the related disease is diabetes.

As use herein, the term "bioactive compound" means any substance other than the LTA of the invention which has biological activity related to its ability to modulate one or more metabolic processes which results in the promotion of better health conditions for a subject. The bioactive compound may interact with the LTA of the invention in order to improve the properties or effects of the LTA, or may interact with the subject metabolism assisting the LTA of the invention in the treatment and/or prevention of obesity, overweight or related diseases, preferably diabetes.

As explained above, due to the capacity of the LTA of the invention of reducing the fat, it can be used for the treatment and/or prevention of obesity, overweight or related diseases such as diabetes.

Thus, in another aspect, the present invention relates to the LTA of the invention or the composition of the invention, together with all their particular embodiments as disclosed previously, for use as a medicament.

The term "medicament" as used herein means a pharmaceutical composition suitable for administration of the pharmaceutically active compound to both human and non-human subjects. Such pharmaceutical compositions, or pharmaceutical formulations, are defined as those which incorporate the LTA of the present invention, combined with, at least one pharmaceutically acceptable excipient and/or carrier. Pharmaceutically acceptable excipients and/or carriers used in the present invention are known in the prior art to experts in the art, and have been disclosed in previous paragraphs.

The medicament is, preferably, administered in a therapeutically effective dose. A therapeutically effective dose refers to an amount of the LTA of the invention to be used in the composition applied such that it prevents, ameliorates or treats the symptoms accompanying a disease or disorder referred to in this specification. Therapeutic efficacy and toxicity of a given drug can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

In another aspect, the present invention relates to the LTA of the invention, or the composition of the invention, together with all their particular embodiments as disclosed previously, alone or in combination with each other, for use in the treatment and/or prevention of obesity, overweight or a related disease selected from the group consisting of diabetes, metabolic syndrome, hypertension, hyperglycaemia, inflammation, type-2 diabetes, cardiovascular disease, hypercholesterolemia, hormonal disorders and infertility.

The term "treatment" as used herein refers to the treatment of a disease or medical condition in a patient, preferably human patient, which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The term "prevention" as used herein means inhibition of the occurrence of the obesity (body weight gain), namely, administration of the LTA of the present invention before the onset of the obesity condition. This means use of the LTA of the present invention as a preventive drug to thereby prevent body weight gain, or to thereby prevent the disease which may be induced by the body weight gain.

The term "obesity" in general refers to an abnormal or excessive fat accumulation that presents a risk to health, being "overweight" a level of obesity. A crude population measure of obesity in adults is the body mass index (BMI), a person's weight (in kilograms) divided by the square of his or her height (in meters). The term "obesity" is herein adopted to describe a condition characterized by, preferably, a BMI ≥ 30 kg/m² while the term "overweight" is herein adopted to describe a condition characterized by, preferably, a BMI ≥ 25 kg/m² but < 30 kg/m². For adolescents, "obesity" refers to a condition characterized by two standard deviations body mass index for age and sex from the World Health Organization (WHO) growth reference for school-aged children and adolescents. The terms "obesity" and "overweight" thus imply a medical indication for treatment.

For the purposes of the present disclosure, the terms "related or associated diseases/disorders" and "diseases/ disorders caused by overweight and/or obesity" comprise those disease or disorders which obesity or overweight is a risk factor of suffering them.

The present disclosure also describes a method of reducing fat accumulation in a subject, preferably a mammal, more preferably a human, comprising administering to the subject an effective amount of the LTA of the invention or the composition of the invention.

Likewise, the present disclosure also describes a method for the prevention and/or treatment of obesity, overweight or related diseases, preferably diabetes, in a subject, preferably a mammal, more preferably a human, comprising administering to the subject an effective amount of the LTA of the invention or the composition of the invention.

Analogously, the present disclosure also describes the use of the LTA of the invention in the manufacture of a pharmaceutical composition (or medicament) for reducing fat accumulation in a subject, or for the prevention and/or treatment of obesity, overweight or related diseases in a subject, preferably, said subject is a mammal, more preferably a human.

All the particular embodiments disclosed for the LTA of the invention and the composition of the invention in previous paragraphs, are applicable to the methods or the uses disclosed in the previous paragraphs.

The present invention also encompasses non-therapeutic uses of the LTA of the invention, or the composition of the invention, and all its particular embodiments alone or in combination each other. Thus, in another aspect, the present invention relates to the non-therapeutic use of the LTA of the invention, or the composition of the invention, for body fat reduction. The particular embodiments relating to the LTA of the invention or to the composition of the invention are applicable to the present inventive aspect. The LTA of the invention, or the composition of the invention, can also be used for non-therapeutic fat reduction in non-obese subjects, i.e. "normal-weight" subjects having a BMI < 25 kg/m² or "overweight" subjects as defined above and without any obesity-associated health implications. Such use is exclusively for aesthetic or cosmetic reasons (cosmetic use) and not based on a medical indication. Also, body fat reduction and/or maintenance of body weight in non-obese subjects might involve body weight reduction and/or maintenance. The cosmetic product will uniquely have a cosmetic effect in the subject who uses it related to body fat reduction. The term "cosmetic effect" is explained below. Analogously, the present invention relates to a non-therapeutic method for body fat reduction comprising administering the LTA of the invention, or the composition of the invention (comprising all its particular embodiments alone or in combination each other) to non-obese subjects.

In another aspect, the present invention relates to the use of the invention, or the composition of the invention, for the elaboration of a food or feed product. The terms "food" and "feed product" have been defined previously herein and are applicable to the present inventive aspect. The process and methods for elaborating foods or feed products are widely known in the state of the art.

In another aspect, the present invention relates to a process for obtaining the LTA of the invention, hereinafter "process of the invention", comprising the following steps:
(a) cultivating a *Bifidobacterium* in excess of sugars as carbon source, and
(b) isolating the LTA from the cell wall of the bacterium.

In a first step, the process for obtaining the LTA of the invention comprises cultivating a *Bifidobacterium* in excess of sugars as carbon source.

In the context of the present invention, "carbon source" refers to the molecules used by an organism as the source of carbon for building its biomass. In the process for obtaining the LTA of the invention, the carbon source used is sugars. Sugars is a term referring to a broad category of all mono- and disaccharides: the simplest carbohydrates. Monosaccharides include glucose, galactose and fructose, and disaccharides include sucrose, lactose, maltose and trehalose. Therefore, in the process for obtaining the LTA of the invention, the sugar may be selected from the list consisting of glucose, galactose, fructose, sucrose, lactose, maltose and trehalose. In a more particular embodiment, the sugar is glucose.

The conditions for carrying out the culture of *Bifidobacterium* are widely known in the state of the art. *Bifidobacterium* can be cultured using growth media such as Man, Rogosa and Sharpe medium (MRS medium), composed by peptone (1%), meat extract (0,8%), yeast extract (0,4%), glucose (2%), dipotassium hydrogen phosphate (0,2%), sodium acetate trihydrate (0,5%) triammonium citrate (0,2%) magnesium sulfate (0,02%) heptahydrate (0,005%) and supplemented with 1 mL/L polysorbate (Tween 80). Bifidobacteria of human origin may be cultured in anaerobic conditions, at 36-38ºC, and pH 6.5-7.0.

In a second step, the process for obtaining the LTA of the invention comprises the isolation of the LTA. The isolation of the LTA from *Bifidobacterium* may be done at any time that *Bifidobacterium* biomass growth occurs. Thus, the isolation may be done from the end of the lag phase and during all the phases the culture. The isolation of the LTA from the *Bifidobacterium* biomass comprises the extraction and recovery of the LTA.

The extraction and recovery of the LTA from Bifidobacteria can be conducted by techniques known to those skilled in the art and the detection thereof can be achieved by conventional analytical methodologies including serology (Huub J. M. Op Den Camp et al. 1985. J. Gen Microbiol., 131 (3), 661-668), and biochemical determinations such as molybdenum blue test to detect phosphate and orcinol-sulfuric acid reaction for hexose quantification.

In a particular embodiment of the process for obtaining the LTA of the invention, the *Bifidobacerium* is *Bifidobacterium animalis,* preferably *Bifidobacterium animalis* subsp. *lactis,* more preferably *Bifidobacterium animalis* subsp. *lactis* CECT 8145.

Alternatively, the Bifidobacterium may be *Bifidobacterium longum,* preferably, *Bifidobacterium longum* CECT 7347.

Finally, the present disclosure also relates to the LTA obtained by the process of the invention as indicated above.

### Medical uses of the LTA from Bifidobacterium cultured in excess of sugars as carbon source

The present invention shows that the LTA from *Bifidobacterium* cultured in excess of sugars as carbon source, preferably glucose, leads to the reduction of fat in *C*. *elegans* (see examples). In addition, the present invention demonstrates that the level of fat reduction of the LTA from *Bifidobacterium* cultured in excess of sugars as carbon source (i.e. the glucose is not a limiting nutrient throughout all the phases of the culture, namely lag phase, exponential phase and stationary phase) is surprisingly higher than that of LTAs obtained from other bacteria genus such as *Lactobacillus* and *Bacillus* bacterial strains (see Table 1 of the examples). Furthermore, examples shown also evidence that when the LTA is obtained from the specific strain *Bifidobacterium animalis* subsp. *lactis* (CECT8145), also called in the present invention "BPL1" or "BPL0001", leads to the highest level of fat reduction among all the strains tested herein (see Table 1 of the examples).

Therefore, another aspect of the present invention relates to a lipoteichoic acid (LTA) from Bifidobacteria cultured in excess of sugars as carbon source, for use as a medicament, wherein the structure of the LTA is the following: 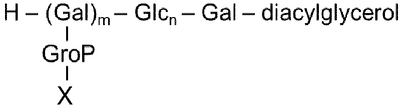 wherein
- GroP is glycerophosphate,
- Gal is galactofuranan,
- each X is hydrogen (H) or Alanine (Ala),
- the number of molecules of Alanine is m or m-p, being m the number of repeating units of Gal and p the number of units of Gal in which X is hydrogen,
- Glc is glucan,
- m is the number of repeating units of Gal and is between 10 and 20, preferably, between 11 to 18, and
- n is the number of repeating units of Glc and is between 5 and 20, preferably, between 8 and 12.

The terms "medicament", "treatment" and "prevention", as well as its particular embodiments, have been defined in previous paragraphs and are applicable to the present aspect of the invention.

As explained in previous paragraphs, the extraction and recovery of the LTA from Bifidobacteria can be conducted by techniques known to those skilled in the art and the detection thereof can be achieved by conventional analytical methodologies including serology (J Huub J.M. OP DEN CAMP et al. 1985, Gen Microbiol., 131 (3), 661-668), and biochemical determinations such as molybdenum blue test to detect phosphate and orcinol-sulfuric acid reaction for hexose quantification.

In a particular embodiment, the invention relates to a LTA from Bifidobacteria cultured in excess of sugars as carbon source for use in the treatment and/or prevention of obesity, overweight or related diseases selected from the group consisting of diabetes, metabolic syndrome, hypertension, hyperglycaemia, inflammation, type-2 diabetes, cardiovascular disease, hypercholesterolemia, hormonal disorders and infertility, wherein the structure of the LTA is the following: 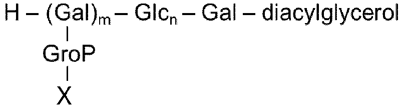 wherein
- GroP is glycerophosphate,
- X is Alanine (Ala) or Hydrogen (H),
- Gal is galactofuranan,
- each X is independently selected from hydrogen and Alanine (Ala),
- the number of molecules of Alanine is m or m-p, being m the number of repeating units of Gal and p the number of units of Gal in which X is hydrogen,
- Glc is glucan,
- m is the number of repeating units of Gal and is between 10 and 20, preferably, between 11 to 18, and
- n is the number of repeating units of Glc and is between 5 and 20, preferably, between 8 and 12.

The expression "cultured in excess of sugars as carbon source" has been explained in the beginning of the present description. The sugar may be selected from the list consisting of glucose, galactose, fructose, sucrose, lactose, maltose and trehalose.

The terms "obesity", "overweight", and "related diseases" have been defined previously in the present description and its definitions are applicable to the present aspects of the invention.

The LTA from Bifidobacteria cultured in excess of sugars as carbon source for use as a medicament and for use in the treatment and/or prevention of obesity, overweight or related diseases, preferably, the related disease is diabetes:
- the molar ratio Alanine/glucose is at least 0.5, 0.6, 0.7, 0.8, 0.9 or 1.0, and the molar ratio glycerol phosphate/glucose is at least 1.2, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0 or 12.5, preferably, the molar ratio glycerol phosphate glucose is 12.6; and/or
- the Alanines are L-Alanine, D-Alanine or a combination of L- and D- Alanine.

Other changes in the proportion/ratio of components of LTA isolated from Bifidobacteria cultured in excess of sugars as carbon sources, preferably glucose, relates to the number of galactoses, glyrecol and phosphorous molecules. Thus, the molar ratio Galactose/glucose is at least 0.8, the mol ratio Glycerol/glucose is at least 1.0, and/or the molar ratio phosphorous/glucose is at least 5.0, 10.0, 15.0, 20.0 or 25.0, preferably the molar ratio phosphorous/glucose is 26.0, more preferably, 26.09 The meaning of the term "molar ratio" has been explained previously in the present description.

In both the LTA from Bifidobacteria cultured in excess of sugars as carbon source, preferably glucose, for use as a medicament and for use in the treatment and/or prevention of obesity, overweight or related diseases, the LTA is obtained from *Bifidobacterium animalis,* preferably from *Bifidobacterium animalis* subsp. *lactis,* more preferably from the strain *Bifidobacterium animalis* subsp. *lactis* CECT 8145.

In particular, the LTA from Bifidobacteria cultured in excess of sugars as carbon source, preferably the sugar is glucose, is obtained from *Bifidobacterium longum,* preferably *Bifidobacterium longum* CECT 7347.

The strains *Bifidobacterium animalis* subsp. *lactis* CECT 8145 and *Bifidobacterium longum* CECT 7347 have been defined previously herein.

In another preferred embodiment, the LTA from Bifidobacteria is heat-treated or lyophilized. The terms "heat-treated" and "lyophilized" has been defined previously.

The present disclosure refers to a method of reducing fat accumulation in a subject, preferably mammal, more preferably human, comprising administering to the subject an effective amount of the LTA from Bifidobacteria. The LTA is obtained from *Bifidobacterium animalis,* preferably from *Bifidobacterium animalis* subsp. *lactis,* more preferably from the strain *Bifidobacterium animalis* subsp. *lactis* CECT 8145, and/or the LTA is heat-treated or lyophilized.

Analogously, within the scope of the medical uses, the present disclosure also relates to the use of the LTA from *Bifidobacterium* cultured in excess of sugars as carbon source in the manufacture of a pharmaceutical composition for reducing fat accumulation in a subject, or for the prevention and/or treatment of obesity, overweight or related diseases in a subject, preferably, said subject is a mammal, more preferably a human.

All the particular embodiments disclosed for the medical uses of the LTA from *Bifidobacterium* cultured in excess of sugars as carbon source, and the composition comprising it, are applicable to the methods or the uses disclosed in the previous paragraphs.

### Non-therapeutic uses of the LTA from Bifidobacteria cultured in excess of sugars as carbon source

As explained above, the LTA from Bifidobacteria cultured in excess of sugars as carbon source, preferably glucose, can also be used for non-therapeutic fat reduction in non-obese subjects, i.e. "normal-weight" subjects having a BMI < 25 kg/m² or "overweight" subjects as defined above and without any obesity-associated health implications. Such use is exclusively for aesthetic or cosmetic reasons (cosmetic use) and not based on a medical indication. Also, body fat reduction and/or maintenance of body weight in non-obese subjects might involve body weight reduction and/or maintenance.

Thus, another aspect of the present invention relates to a non-therapeutic use (or cosmetic use) of the LTA from Bifidobacteria cultured in excess of sugars as carbon source for body fat reduction, wherein the structure of the LTA is the following: 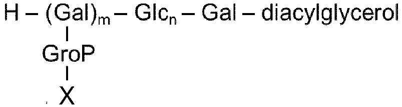 wherein
- GroP is glycerophosphate,
- X is Alanine (Ala) or Hydrogen (H),
- Gal is galactofuranan,
- each X is independently selected from hydrogen and Alanine (Ala),
- the number of molecules of Alanine is m or m-p, being m the number of repeating units of Gal and p the number of units of Gal in which X is hydrogen,
- Glc is glucan,
- m is the number of repeating units of Gal and is between 10 and 20, preferably, between 11 to 18, and
- n is the number of repeating units of Glc and is between 5 and 20, preferably, between 8 and 12.

In the non-therapeutic use of LTA from Bifidobacteria cultured in excess of sugars as carbon source, preferably glucose:
- the molar ratio Alanine/glucose may be at least 0.5, 0.6, 0.7, 0.8, 0.9 or 1.0, and the molar ratio glycerol phosphate/glucose may be at least 1.2, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0 or 12.5, preferably, the molar ratio glycerol phosphate glucose may be 12.6; and/or
- the Alanines may be L-Alanine, D-Alanine or a combination of L- and D- Alanine.

The meaning of the term "molar ratio" has been explained previously in the present description.

Other changes in the proportion/ratio of components of LTA isolated from Bifidobacteria cultured in excess of sugars as carbon sources, preferably glucose, relate to the number of galactoses, glycerol and phosphorous molecules. Thus, the molar ratio Galactose/glucose may be at least 0.8, the mol ratio Glycerol/glucose may be at least 1.0, and/or the molar ratio phosphorous/glucose may be at least 5.0, 10.0, 15.0, 20.0 or 25.0, preferably the molar ratio phosphorous/glucose may be 26.0, more preferably, 26.09.

In the non-therapeutic use of LTA from Bifidobacteria cultured in excess of sugars as carbon source, preferably glucose, alone or in combination with the previous particular embodiments, the LTA from Bifidobacteria cultured in excess of sugars as carbon source, preferably glucose, may be obtained from *Bifidobacterium animalis,* preferably from *Bifidobacterium animalis* subsp. *lactis,* more preferably from the strain *Bifidobacterium animalis* subsp. *lactis* CECT 8145. Alternatively, the LTA from Bifidobacteria cultured in excess of sugars as carbon source, preferably the sugar may be glucose, may be obtained from *Bifidobacterium longum,* preferably *Bifidobacterium longum* CECT 7347. The strains *Bifidobacterium animalis* subsp. *lactis* CECT 8145 and *Bifidobacterium longum* CECT 7347 have been defined previously herein.

In another particular embodiment of the non-therapeutic use of LTA from Bifidobacteria cultured in excess of sugars as carbon source, preferably sugar, the LTA is heat-treated or lyophilized. The terms "heat-treated" and "lyophilized" have been defined previously.

The cosmetic product will uniquely have a cosmetic effect in the subject who uses it related to body fat reduction.

The term "cosmetic effect" as used herein refers to the desired advantageous impact of the LTA from Bifidobacteria cultured in excess of sugars as carbon source, preferably glucose, with regard to appearance, which is associated with loss or maintenance of body fat, and preferably an enhancement of body shape and definition. It is to be understood that the non-therapeutic cosmetic treatment of a subject with the LTA from Bifidobacteria cultured in excess of glucose is for aesthetic reasons only and is exclusively accomplished in subjects that do not exhibit an amount of body fat that significantly increases health risks. Non-therapeutic, cosmetic treatment with the LTA from Bifidobacteria cultured in excess of glucose may also induce loss of weight and/or serve for weight control in order to prevent a (non-pathological) body fat accumulation and/or gain of weight.

In another aspect, the present invention refers to the use of the LTA from Bifidobacteria cultured in excess of sugars as carbon source, preferably glucose, for the elaboration of a food or feed product, wherein the structure of the LTA is the following: 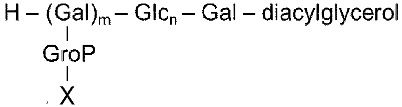 wherein
- GroP is glycerophosphate,
- X is Alanine (Ala) or Hydrogen (H),
- Gal is galactofuranan,
- each X is independently selected from hydrogen and Alanine (Ala),
- the number of molecules of Alanine is m or m-p, being m the number of repeating units of Gal and p the number of units of Gal in which X is hydrogen,
- Glc is glucan,
- m is the number of repeating units of Gal and is between 10 and 20, preferably, between 11 to 18, and
- n is the number of repeating units of Glc and is between 5 and 20, preferably, between 8 and 12.

In the use of the LTA from Bifidobacteria cultured in excess of sugars as carbon source, preferably glucose, for the elaboration of a food product or feed product:
- the molar ratio Alanine/glucose may be at least 0.5, 0.6, 0.7, 0.8, 0.9 or 1.0, and the molar ratio glycerol phosphate/glucose may be at least 1.2, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0 or 12.5, preferably, the molar ratio glycerol phosphate glucose may be 12.6; and/or
- the Alanines may be L-Alanine, D-Alanine or a combination of L- and D- Alanine.

The meaning of the term "molar ratio" has been explained previously in the present description.

Other changes in the proportion/ratio of components of LTA isolated from Bifidobacteria cultured in excess of sugars as carbon sources, preferably glucose, relate to the number of galactoses, glyrecol and phosphorous molecules. Thus, the molar ratio Galactose/glucose may be at least 0.8, the mol ratio Glycerol/glucose may be at least 1.0, and/or the molar ratio phosphorous/glucose may be at least 5.0, 10.0, 15.0, 20.0 or 25.0, preferably the molar ratio phosphorous/glucose may be 26.0, more preferably, 26.09.

The terms "food" or "feed product" has been defined in previous paragraphs. Examples of food or feed products include, without limiting to, functional foods, probiotics, beverages, symbiotic or synbiotic, dietary supplements and/or nutraceutical incorporating the LTA from Bifidobacteria cultured in excess of sugars as carbon source, preferably glucose, and/or extracts comprising the same. In principle, the food is not limited in terms of form and type. For example, the food product can be in liquid, powdery, or solid form. The food product referred to in the present invention may be for human or non-human animal consumption, including infant nutrition, feed and pet food. Food products of the invention comprise functional foods commonly known in the art. They may further include different nutrients, vitamins, electrolytes, flavours, colouring agents, pectic acid and salts thereof, alginic acid and salts thereof, organic acid, protective colloidal thickeners, pH modifiers, stabilizers, preservatives, glycerine, alcohol, a carbonating agent used for carbonated drinks (so called 'sparkling drinks'), or the like.

Examples of foodstuffs possibly containing the LTA from Bifidobacteria cultured in excess of glucose may include meat, sausages, bread, chocolate, candy, snacks, confections, pizza, ramen noodles, other noodles, gum, dairy products including ice cream, various soups, beverages, teas, drinks, milk, feed, etc.

In a particular embodiment of the use of the LTA from Bifidobacteria cultured in excess of sugars as carbon source, preferably glucose, for the elaboration of a food or feed product, alone or in combination with the previous particular embodiments, the food or feed product is a nutritional supplement. The term "nutritional supplement" has been defined previously in the present description.

In the use of the LTA from Bifidobacteria cultured in excess of sugars as carbon source, preferably glucose, for the elaboration of a food or feed product, alone or in combination with the previous particular embodiments, the LTA ma be obtained from *Bifidobacterium animalis,* preferably from *Bifidobacterium animalis* subsp. *lactis,* more preferably from the strain *Bifidobacterium animalis* subsp. *lactis* CECT 8145. Alternatively, the LTA from Bifidobacteria cultured in excess of sugars as carbon source, preferably glucose, is obtained from *Bifidobacterium longum,* preferably *Bifidobacterium longum* CECT 7347. The strains *Bifidobacterium animalis* subsp. *lactis* CECT 8145 and *Bifidobacterium longum* CECT 7347 have been defined previously herein.

In another particular embodiment of the use of the LTA from Bifidobacteria cultured in excess of sugars as carbon source, preferably glucose, for the elaboration of a food or feed product, alone or in combination with the previous particular embodiments, the LTA is heat-treated or dried including lyophilization. The terms "heat-treated" and "lyophilisation" have been defined previously herein.

The present disclosure relates to a process for obtaining the LTA from Bifidobacteria, comprising the following steps
(a) cultivating a *Bifidobacterium* in excess of sugars as carbon source, and
(b) isolating the LTA from the cell wall of the bacterium.

The terms "carbon source" and "sugars", as well as their particular embodiments, have been already defined in previous paragraphs and apply to the present process. In tthe process for obtaining the LTA from Bifidobacteria, the sugar may be selected from the list consisting of glucose, galactose, fructose, sucrose, lactose, maltose and trehalose.

The conditions for carrying out the culture of *Bifidobacterium* and the isolation of the LTA from said *Bifidobacerium* are widely known in the state of the art and have been previously explained in the present description.

In the process for obtaining the LTA, the *Bifidobacerium* may *Bifidobacterium animalis,* preferably *Bifidobacterium animalis* subsp. *lactis,* more preferably *Bifidobacterium animalis* subsp. *lactis* CECT 8145. Alternatively, in the process for obtaining the LTA, the Bifidobacteria may be *Bifidobacterium longum,* preferably *Bifidobacterium longum* CECT 7347. The strains *Bifidobacterium animalis* subsp. *lactis* CECT 8145 and *Bifidobacterium longum* CECT 7347 have been defined previously herein.

The present disclosure also relates to an LTA obtained by a process comprising (a) cultivating a *Bifidobacterium* in excess of sugars as carbon source, preferably glucose, and (b) isolating the LTA from the cell wall of the bacterium.

### DESCRIPTION OF THE DRAWINGS

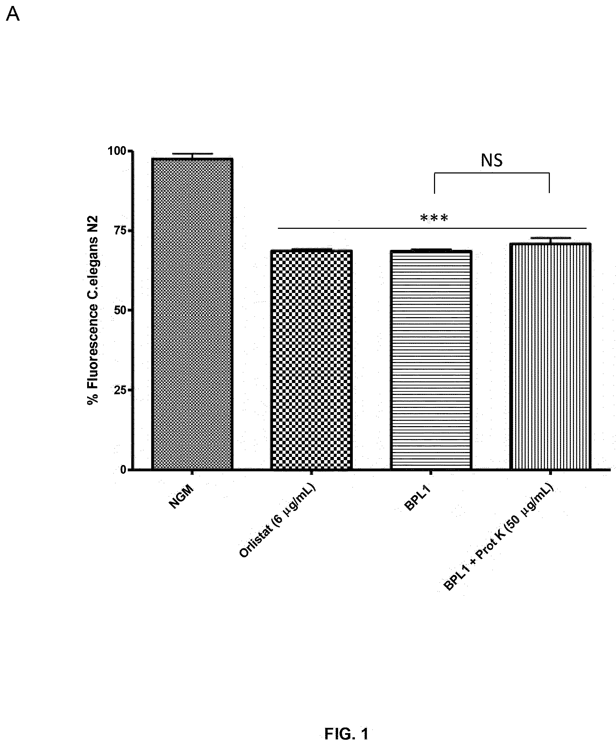

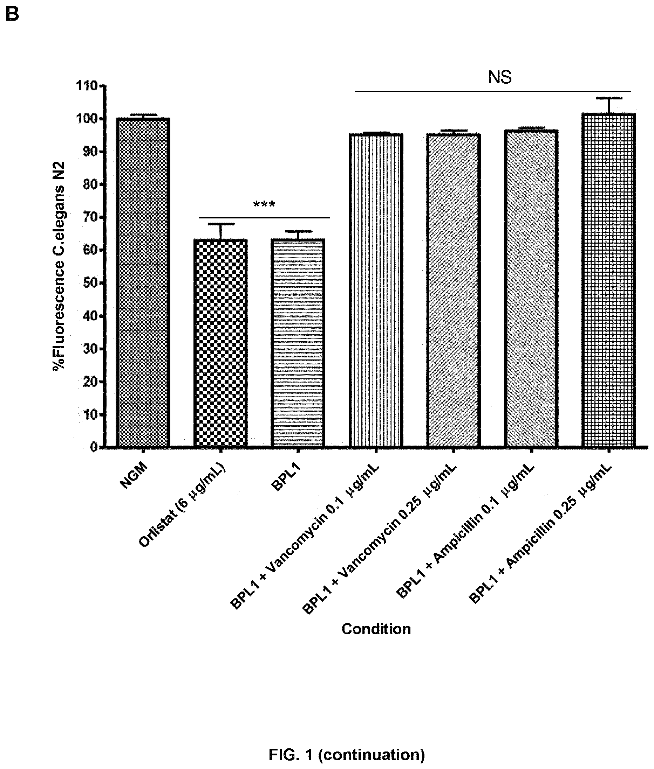

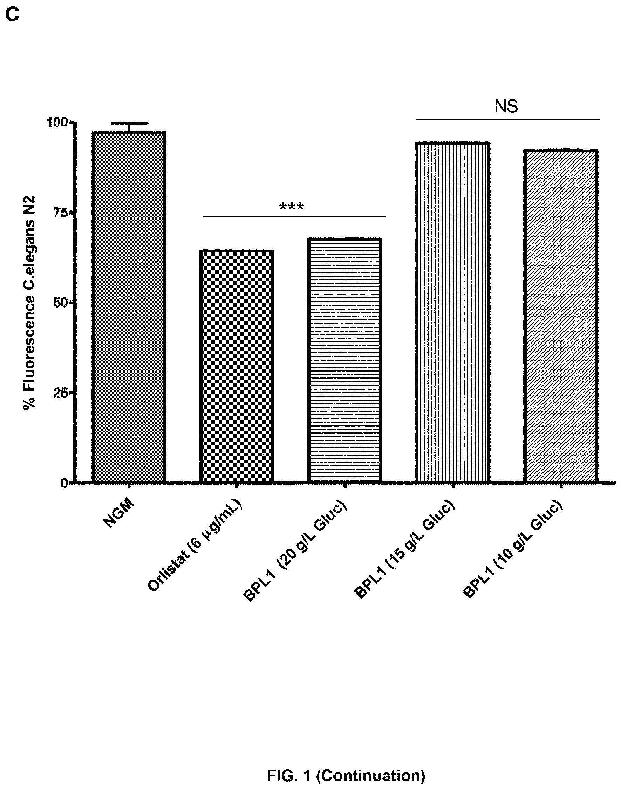

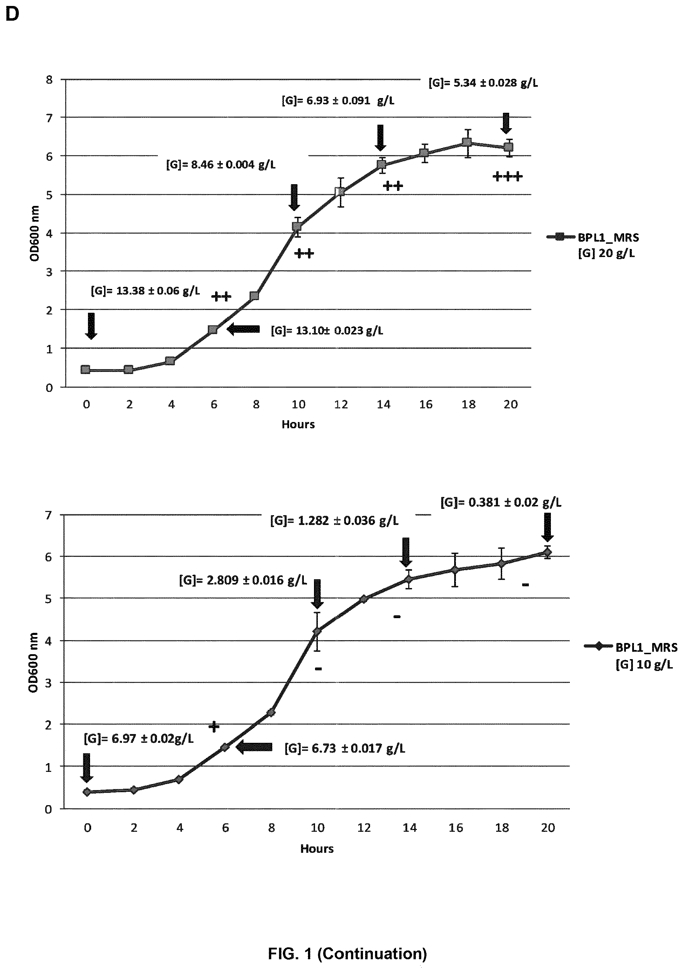

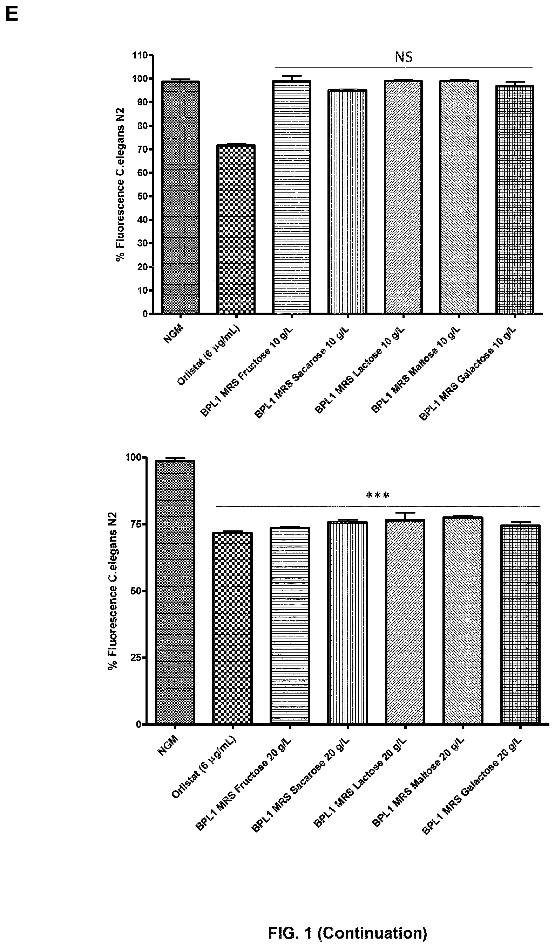

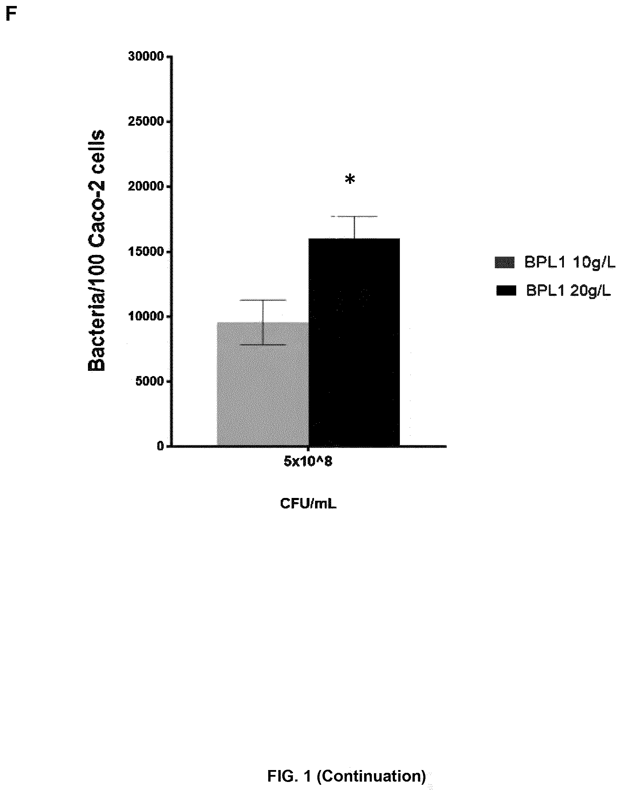

**Figure 1****.** *B. animalis* subsp. *lactis* BPL1 functional activity relies on cell envelop component/s. A) Protease treatment of BPL1 cells. B) BPL1 cells treated with vancomycin and ampicillin at doses below their MIC. C) Glucose restriction (15 g/L, 10 g/L) conditions at the culture media (MRS-Cys). D) Cell growth curves of BPL1 strain obtained by quantification of OD at 600 nm in standard MRS-Cys culture medium (20 g/L glucose) or MRS-Cys at low glucose (10 g/L). Glucose levels were estimated at different times along the growth curve. Data are mean ± sd. and were calculated from two biological independent experiments. E) Restriction of Fructose, saccharose, lactose, maltose, or galactose (10 g/L) at the culture media (MRS-Cys) F) Adherence to Caco-2 epithelial cells. BPL1 cultures at 5x 10⁸ CFU/mL grown in glucose 10 g/L (restricted) or 20 g/L (standard) were used in the adhesion assays. Data are mean ± sd of three independent experiments. For A), B), C), percentage of fluorescence in bacterial-fed nematodes (Wild-type-strain N2) is represented. Nile red was quantified at young adult stage. Orlistat (6 µg/mL) was used as positive control. Data are mean ± sd and were calculated from two independent biological experiments. **P*<0.05; ***P*<0.01; ****P*<0.001, NS: not significant.
**Figure 2****.** Crude cell wall fraction from BPL1 exerts fat-reducing activity. Preparations of cell wall fraction of BPL1, of cells grown in standard conditions (MRS-Cys with 20 g/L of glucose) and low glucose (10 g/L). Percentage of fluorescence in cell wall fraction-fed nematodes (Wild-type-strain N2) is represented. Nile red was quantified at young adult stage. Orlistat (6 µg/mL) was used as positive control. Data are mean ± sd. and were calculated from two independent biological experiments. ***P*<0.01; ****P*<0.001, NS: not significant.

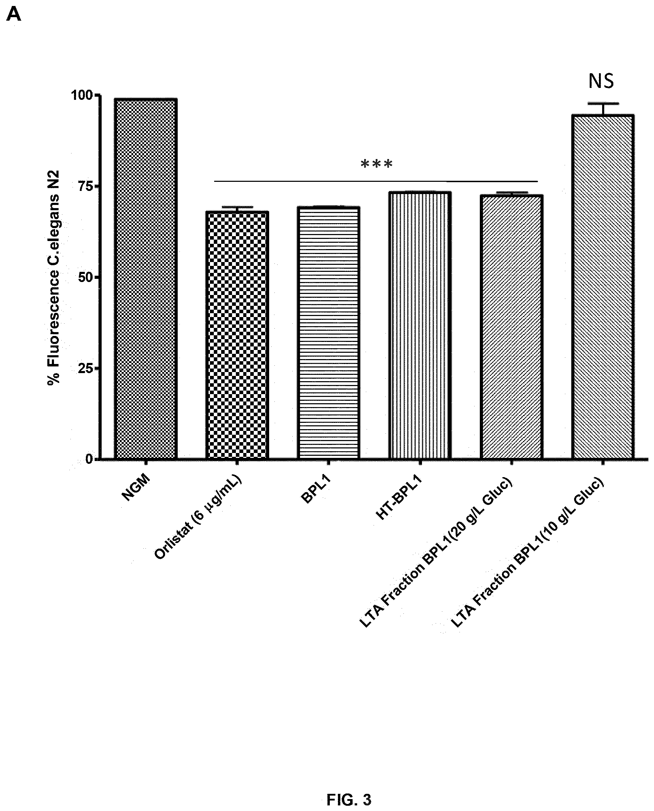

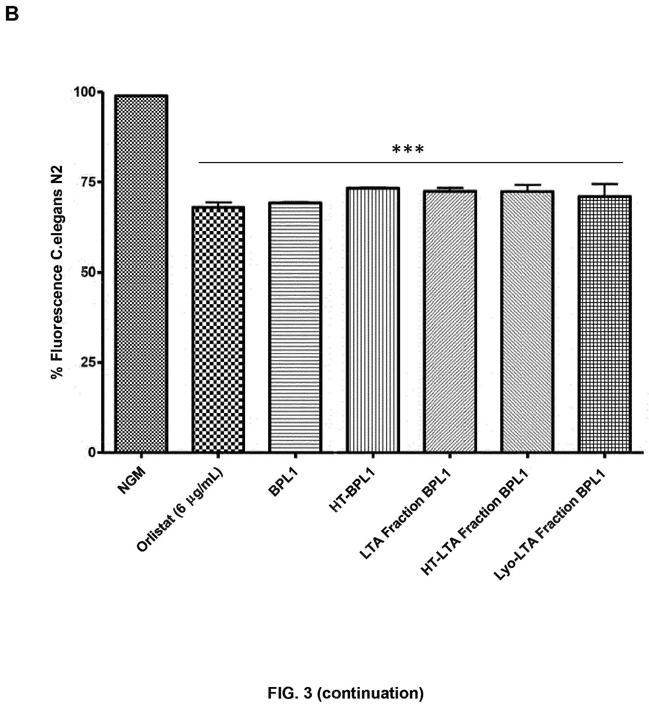

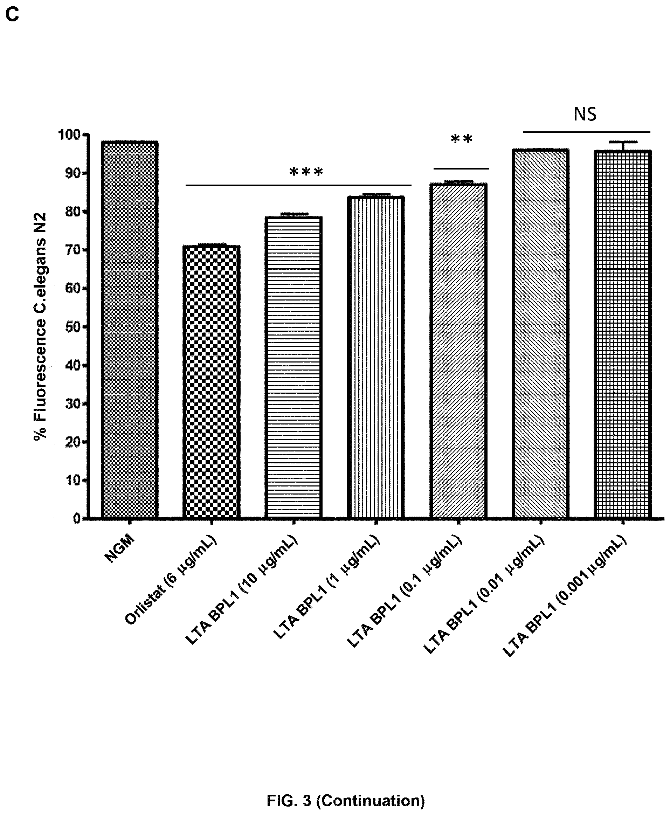

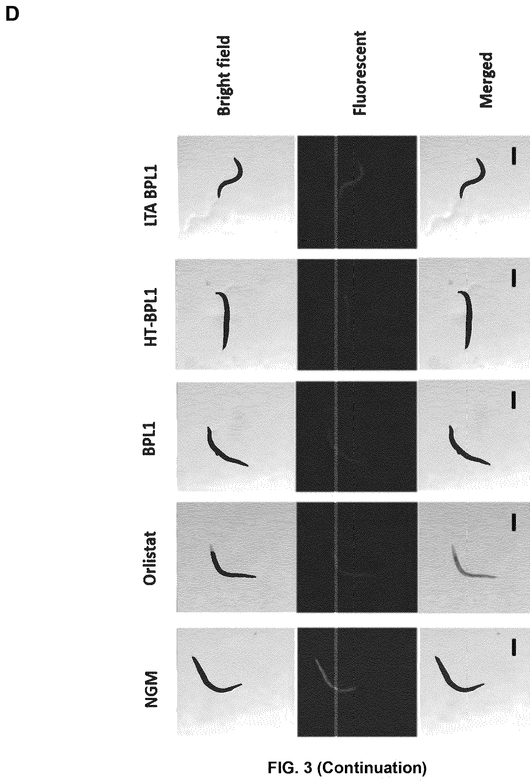

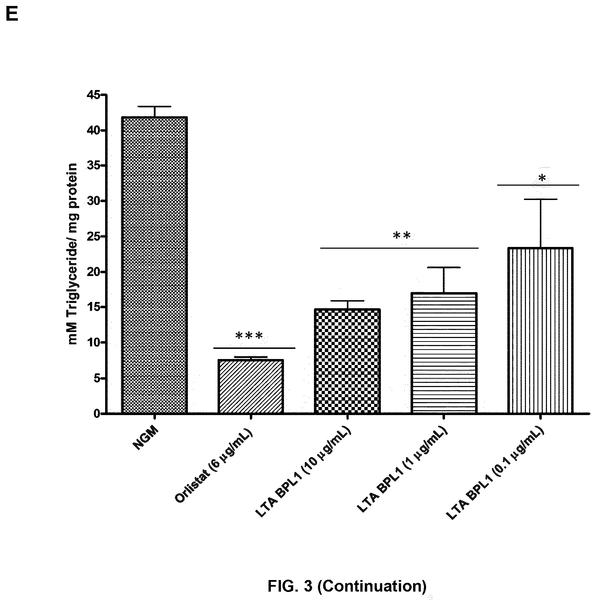

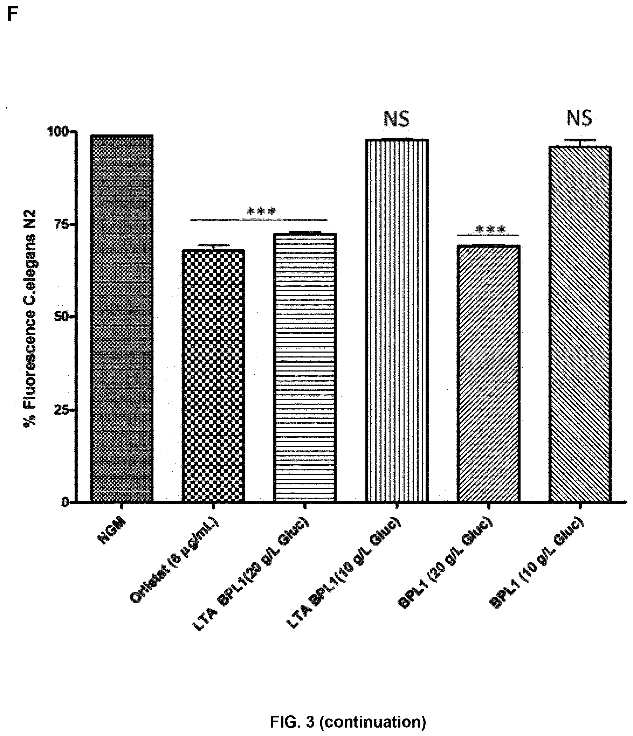

**Figure 3****.** Lipoteichoic acid (LTA) from BPL1 strain demonstrates fat reducing activity and preserves this capacity under different treatments. A) LTA fraction obtained from BPL1 in standard MRS-Cys (20 g/L) and from BPL1 cells grown in low glucose MRS-Cys (10 g/L). B) Heating or lyophilization of LTA fraction from BPL1 cells did not impact on its functionality. C) Fat-reducing effect of purified LTA at different doses. D) Representative images of Nile red staining of lipid content in live young adult *C*. *elegans* in a *Wild-type* N2 animal under fluorescence microscopy. Nematodes were fed with BPL1 cells, heat-treated BPL1 cells (HT-BPL1) or LTA. Scale bar 250 µm. Original image taken by the authors for this paper with a Nikon-SMZ18 Fluorescence Stereomicroscope. E) Quantification of triglyceride content (mM TG/mg protein) in C. elegans fed with purified LTA from BPL1. F) Purified LTA obtained from BPL1 in standard MRS-Cys (20 g/L) and from BPL1 cells grown in low glucose MRS-Cys (10 g/L). BPL1 cells grown in excess or restriction of glucose were included. For A), B), C), F) percentage of fluorescence in bacterial and LTA-fed nematodes (Wild-type-strain N2) is represented. Nile red was quantified at young adult stage. Orlistat (6 µg/mL) was used as positive control. Data are mean ± sd. and were calculated from two biological independent experiments. ***P*<0.01; ****P*<0.001; NS: not significant.

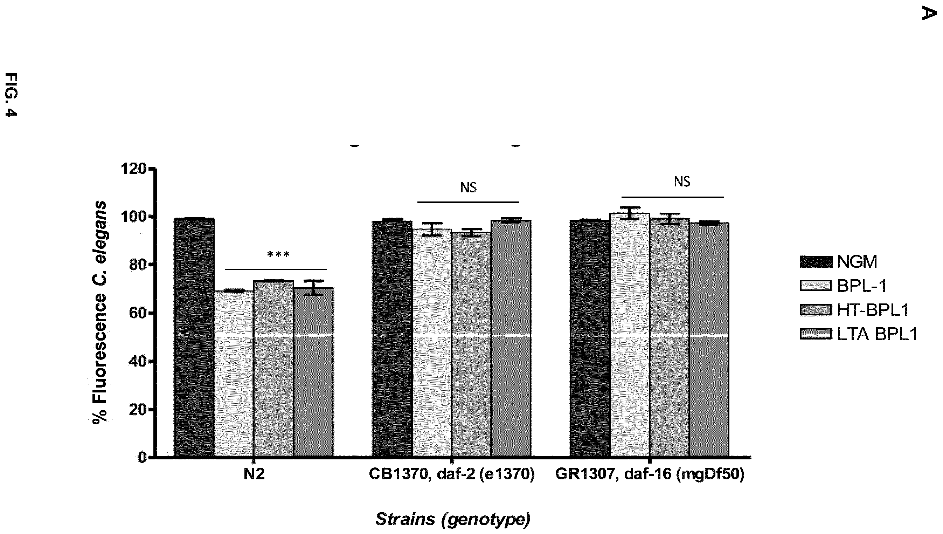

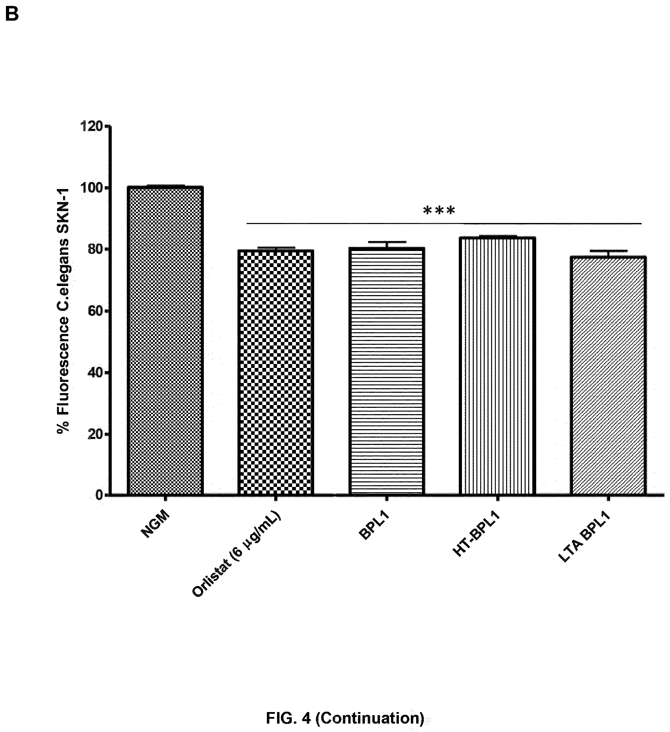

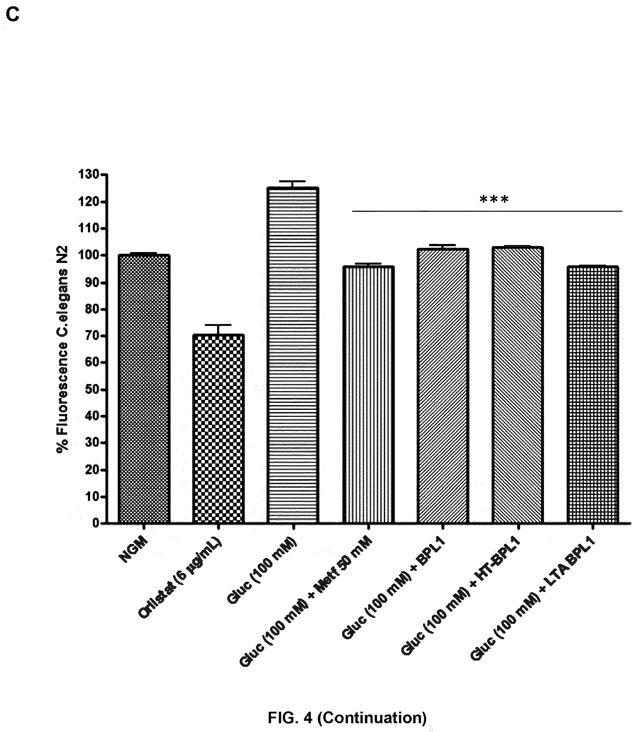

**Figure 4****.** Lipoteichoic acid (LTA) from BPL1 strain requires the Insulin-like signaling pathway (IGF-I) to exert its fat reducing effect, and has functional activity in hyperglycemic conditions. A) Feeding worms with LTA in mutant *daf-2* and *daf-16* strains; the same as with the live BPL1 cells and the heat-treated BPL1 cells. B) Fat content in *C. elegans* mutant for SKN-1 transcription factor treated with LTA from BPL1. C) Effect of treatments on a *C. elegans* hyperglycemic model. Nematodes of wild-type strain N2 grown in high glucose (100 mM). Metformin was used as positive control. For A), B), C) percentage of fluorescence in LTA-fed nematodes (Wild-type-strain N2, GR1307, *daf-16* (mgDf50), CB1370, *daf-2* (e1370), or LG333 *Skn-1* (zu 135)). Nile red staining was quantified at young adult stage. Orlistat (6 µg/mL) was used as positive control. Data are mean ± sd. and were calculated from two independent biological experiments. ****P*<0.001, NS: not significant.
**Figure 5****.** LTA from BPL1 has fat-reducing activity and the mechanical disruption provides slightly more effective LTA. Cells, both alive (BPL1) and disrupted by PANDA homogenizer or Sonication, were included as positive controls. NGM negative control, Orlistat is a fat-reducing drug used as positive control in the assay.
**Figure 6****.** Fat reducing effect in *C. elegans* of LTAs fractions obtained from *B. animalis* subsp. *lactis* BPL1 (CECT 8145). Cells, both alive (BPL1) and heat-treated (HT-BPL1), were included as positive controls. NGM negative control, Orlistat is a fat-reducing drug used as positive control in the assay.
**Figure 7****.** Fat reducing effect in *C. elegans* of LTAs fractions obtained from *B. animalis* subsp- *lactis* BPL1 (CECT 8145) and other *Bifidobacterium, Lactobacillus* and *Bacillus* strains.
**Figure 8****.** Analysis of fat reducing effect in *C. elegans* of purified LTAs obtained from other *Bifidobacterium* strains, *B. longum* ES1 and *B. animalis* BPL1.
**Figure 9****.** NMR spectrum of BPL1-LTA.
**Figure 10****.** Functional evaluation of BPL1 cells obtained at different growth stages and under standard glucose medium (A) or glucose restrictions (B). ***Significant P value <0.001; **Significant P value < 0.01; NS: Not significant.
**Figure 11****.** Purification and characterization of lipoteichoic acid (LTA). (A) Screening for phosphate content of hydrophobic interaction chromatography fractions after N-butanol extraction of BPL1 cell-wall material. (B) SDSPAGE and Alcian blue/silver staining.
**Figure 12****.** MALDI-TOF mass spectrum.

### EXAMPLES

### Example 1: Reduction of fat deposition by lipoteichoic acid (LTA) from Bifidobacterium animalis subsp. lactis CECT 8145 (BPL1)

### I - MATERIAL AND METHODS

### C. elegans strains and maintenance

*Caenorhabditis elegans* N2 wild-type strain (Bristol) and the mutant strains GR1307, *daf-16* (mgDf50), CB1370, *daf-2* (e1370), and LG333 *Skn-1* (zu 135) were provided by *Caenorhabditis* Genetic Center (CGC), University of Minnesota (USA).

The nematode strains were routinely propagated on Nematode Growth Medium (NGM) plates with *Escherichia coli* strain OP50 as a food source at 20 ºC. Worms were synchronized by isolating eggs from gravid adults at 20 ºC, and eggs were hatched in NGM plates. In the experiments for fat quantification, the worms were fed with the different compounds from egg to adult stage.

To induce hyperglycemic conditions, nematodes were grown in NGM plates supplemented with 100 mM of glucose until reaching the young adult stage.

### Bifidobacterium animalis subsp. lactis CECT 8145 (BPL1) culture conditions

*B. animalis* subsp. *lactis* CECT 8145 (BPL1) strain was originally isolated from feces of breastfed healthy babies as previously described (Martorell, P., et al. 2016, J Agric Food Chem 64: 3462-3472). The present study was conducted according to the Helsinki declaration and guidelines for the ethical conduct of medical research involving children. A written informed consent was obtained from the mother after receiving written information. All experimental protocols were approved by our institution committee (Biopolis Biosafety Committee) in accordance with relevant guidelines and regulations. For standard cultivation, bacteria were grown in MRS medium (Peptone from casein, tryptic digest 10 g/L; meat extract 10 g/L; yeast extract 10 g/L; D-glucose 20 g/L; K₂HPO₄ 2 g/L; di-ammonium hydrogen citrate 2 g/L; sodium acetate 5 g/L; MgSO₄ 0.2 g/L; MnSO₄ 0.05 g/L; Tween 80 1 g/L) supplemented with cysteine (Sigma, 0.05% wt/vol)), MRS-Cys, for 18 hours at 37°C in an anaerobiosis atmosphere generated by GasPak^{™} EZ Anaerobe Container System (BD).

*Escherichia coli* OP50 strain was cultured in LB broth (Bacto-tryptone 10 g/L; Bacto-yeast 5 g/L; NaCl 5 g/L) for 18 hours at 37°C. The *E*. *coli* OP50 culture was ready for use in seeding NGM plates.

### Functional evaluation of BPL1 culture supernatant

An overnight culture of BPL1 was obtained, and cells were pelleted by centrifugation at 3220 x *g* for 10 minutes. Supernatant was collected in a new tube and pH was adjusted to 7. Finally, the BPL1 supernatant was filtered using a 0.22 pore size µm filter and tested in a *C. elegans* fat reduction assay. Three different doses (50, 100 and 200 µL/plate) were tested with Orlistat (6 µg/mL) as a positive control.

### DNA isolation from BPL1

DNA from BPL1 cells was isolated from overnight cultures using the High Pure PCR Template Preparation Kit (11796828001, Roche) according to manufacturer instructions. Once DNA was isolated and quantified using Nanodrop spectrophotometer (Thermofischer), three different doses (12.5 µg/plate, 25 µg/plate and 50 µg/plate) were tested on *C. elegans* fat reduction assay by adding directly DNA on the top of NGM agar plates, already seeded with *E. coli* OP50.

### Influence of glucose

To test the influence of glucose on the functional effect of BPL1 cells, glucose concentration was modified in the MRS-Cys medium. BPL1 was grown in MRS-Cys with different concentrations of D-glucose (20, 15 and 10 g/L), maintaining the other ingredients constant (see recipes above). After 18 hours at 37°C in an anaerobic atmosphere generated by GasPak^{™} EZ Anaerobe Container System (BD), bacteria cultured under these glucose conditions were tested in the *C*. *elegans* fat reduction assay. Cells were harvested by centrifugation and washed three times with saline solution Consecutively, concentrated BPL1 treated cells (OD₆₀₀=30) were tested on *C. elegans* fat reduction assay by adding 50 µL on the top of NGM agar plates with *E. coli* OP50*.*

To test the influence of glucose contained in the culture medium on the functional effect of LTA from BPL1, glucose concentration was modified in the MRS-Cys medium. BPL1 was grown in MRS-Cys with different concentrations of D-glucose (20, 15 and 10 g/L), maintaining the other ingredients constant (see recipes above). After 18 horas at 37°C in an anaerobic atmosphere generated by GasPakTM EZ Anaerobe Container System (BD), cells were harvested by centrifugation and washed three times with saline solution and used for LTA isolation and purification.

### Influence of other sugars used as carbon source

The influence of other sugars used as carbon source in the culture of BPL1 strain was tested in modified MRS-Cys medium. D-glucose was replaced by 20g/L and 10 g/L of fructose, saccharose, lactose, maltose or galactose, maintaining the other ingredients constant (see recipes above). After 18 hours at 37°C in an anaerobic atmosphere generated by GasPak^{™} EZ Anaerobe Container System (BD), bacteria cultured under these glucose conditions were tested in the *C. elegans* fat reduction assay as detailed above.

### Influence of antibiotics

BPL1 was also cultured with MRS-Cys medium supplemented with ampicillin or vancomycin, using doses below MIC to *Bifidobacterium* genus. MRS-Cys medium was supplemented with 0.1 µg/mL and 0.25 µg/mL of each antibiotic and BPL1 cells were cultured for 18 hours at 37°C anaerobically. After growth, cells were harvested by centrifugation and washed three times with saline solution. Consecutively, concentrated BPL1 treated cells (OD₆₀₀=30) were tested on *C. elegans* fat reduction assay by adding 50 µL on the top of NGM agar plates with *E. coli* OP50.

### Enzymatic treatments

Proteinase K treatment of BPL1 cells was performed according Gopal et al. [Gopal, P. K., et al. 2001. Int J Food Microbiol 67, 207-216]. Overnight (18 hours, 37°C anaerobically) cultures of BPL1 were treated with Proteinase K (P6556, Sigma). After growth, cells were harvested by centrifugation and washed three times with 50 mM phosphate buffer (pH 7). Stock solution of Proteinase K (1 mg/mL) was prepared in 50 mM phosphate buffer (pH 7). Then, washed cultures were incubated with Proteinase K for 30 minutes at 37°C. After incubation, cells were recovered by centrifugation and washed three times with M9 buffer (KH₂PO₄ 3 g/L; Na₂HPO_{4;} 6 g/L; NaCl 5 g/L; 1 mL 1 M MgSO₄) in order to stop the reaction. Treated cells were concentrated (OD₆₀₀=30) and tested in *C. elegans* for fat reduction by adding 50 µL of cells on the surface of NGM plates with *E. coli* OP50.

### Cellular adhesion assays in Caco-2 cultures

### Cell culture and preparation

Caco-2 cells were placed in Dulbecco's modified Eagle's minimal essential medium DMEM with L-glutamine supplemented with 10% (v:v) fetal bovine serum and 1% (v:v) nonessential amino acids solution. For adhesion assay, the Caco-2 cells were seeded at a concentration of 10⁵ cells/well in 24-well standard tissue culture plates. The cells were maintained for 2 weeks after the confluence, when they were considered to be fully differentiated M. Pinto, S. R.-L., et al. 1983. Biol. Chem, 323- 330.

### Adhesion assay

Overnight cultures of BLP1 were centrifuged to remove culture medium. The bacterial pellet was washed with PBS buffer (NaCl 8 g/L; KCI 0.2 g/L; Na₂HPO₄ 1.15 g/L; KH₂PO₄ 0.2 g/L) and resuspended in cell culture medium and checked for optical density, to give about 1×10⁸, 5x10⁸ or 1x10⁹ cells/mL. Bacterial cells were then incubated with Caco-2 cells for 2 hours under standard conditions. Afterwards, the unattached BPL1 cells were removed by 3-fold washing with PBS. In order to enumerate the attached bacterial cells, monolayers in each well were recovered by pipetting. Mixtures of Caco-2 cells and attached probiotics were plated on MRS-Cys agar. To this end, serial decimal dilutions ranging from 10⁴ to 10⁷ CFU/mL were prepared. Material from each dilution was inoculated into Petri dishes by pouring, using MRS-Cys broth. Bacterial cultures were incubated under anaerobic conditions at 37°C for 48 hours. The bacterial cell density from the stock culture used for the adhesion assay was also estimated. After incubation, the number of adhered bacteria was quantified. Based on the results, the number of adhering bacteria per 100 Caco-2 epithelial cells was calculated.

### Fat reduction assays in C. elegans

*Caenorhabditis elegans* N2 wild-type strain (Bristol) and the mutant strains GR1307, daf-16 (mgDf50), CB1370, daf-2 (e1370), and LG333 Skn-1 (zu 135) were provided by Caenorhabditis Genetic Center (CGC), University of Minnesota (USA). The *C*. *elegans* fat content was measured using the Nile red (Sigma, St. Louis, MO, USA) staining method, following the protocol previously described Martorell, P. et al. 2016 J Agric Food Chem 64, 3462-3472. The dye was added to the surface of NGM plates seeded with OP50 at a final concentration of 0.05 µg/mL. Positive control of the assay was NGM plates with *E. coli* OP50 with Orlistat (6 µg/mL).

Synchronized worms were incubated in these plates for three days until reaching young adult stage. After this period, nematodes were transferred to M9 buffer and the fluorescence was measured using an FP-6200 system (JASCO Analytical Instrument, Easton, MD, USA) with a λₑₓ= 480 nm and λₑₘ= 571 nm. Two experiments were performed per condition to analyze a total of 120 nematodes per condition/treatment.

### Triglyceride (TG) quantification

Total TGs were quantified in nematodes fed with the isolated LTA from BPL1 cultured in excess of glucose using the Triglyceride Quantification Kit (Biovision, Mountain View, CA, USA). Age-synchronized worms were cultured in NGM plates already seeded with E. coli OP50 or NGM plates supplemented with purified LTA (10 µg/mL, 1 µg/mL and 0.1 µg/mL). Worms at young adult stage were then collected and washed using M9 buffer. After worm settling, supernatant was removed, and 400 µL of the triglyceride assay buffer was added to worm pellet. Worms were sonicated with a digital sonifier (Branson Ultrasonics Corp., Danbury, CT, USA) using 4 pulses of 30 s at 10% power. Protein content of each condition was measured using BCA Protein Assay Kit (Thermo Scientific, Rockford, IL, USA). Samples were slowly heated twice at 90°C for 5 min in a thermomixer (ThermoFisher) to solubilize all TG in the solution. After brief centrifugation, aliquots (50 µL/well) were used for the triglyceride assay following the manufacturer instructions. Four different biological replicates were included for each condition in four independent experiments.

### Microscopy analysis

A fluorescent stereomicroscope was used to visualize the Nile-red stained lipid droplets of nematodes under different treatments. Populations of worms incubated from egg to young adult stage in NGM plates with Nile red (0.05 µg/mL) with the same doses of BPL1, HT-BPL1 and LTA BPL1 tested in regular *C*. *elegans* fat reduction assay. Age-synchronized worms were transferred to a new agarose 1% (wt/v) plates and fluorescence was measured in a Fluorescence Stereo microscope (Nikon-SMZ18), equipped with NIS-ELEMENT image software. A total of 30 worms were analyzed per condition. Orlistat (6 µg/mL) was used as positive control.

### Cell wall fraction

BPL1 overnight cultures (100 mL) were boiled for 10 minutes and then centrifuged 14000 x *g* for 8 minutes at 4°C. Pelleted cells were resuspended in 5% (wt/vol) sodium dodecyl sulfate (SDS) and boiled for 25 minutes. Then cells were recovered by centrifugation and resuspended and boiled again in 4% (wt/vol) SDS for 15 minutes. Insoluble material was washed five times with distilled water at 60°C. Afterwards, insoluble cell wall fraction was treated with 2 mg/mL of Pronase (10165921001, Roche) in order to remove covalently attached proteins for 1 hour at 60°C. Cell wall extract was recovered by centrifugation and washed once with distilled water and then insoluble pellet was resuspended in 400 µL of 48% (vol/vol) hydrofluoric acid (HF) (339261-100ML, Sigma) and incubated for 24 hours at 2°C. Cell wall fraction was recovered by centrifugation and washed once with 50 mM Tris-HCl (pH 7) buffer and five times with cold water to eliminate the buffer. After the last wash, the insoluble cell wall containing fraction was normalized at 10 mg/mL using a standard curve of lyophilized *Micrococcus lysodeikticus* (M3770, Sigma) measuring absorption at 206 nm (Schaub, R. E. & Dillard, J. P. 2017. Bio Protoc 7, doi:10.21769/BioProtoc.2438). Finally, cell wall fraction was stored at -20°C. The cell wall fraction was evaluated in *C. elegans* fat reduction assay at a dose of 27.5 µg/mL.

### LTA purification and analysis

The bacteria underwent butanol extraction and hydrophobic interaction chromatography as described previously (Kho, K., and Meredith, T.C. 2018, Journal of Bacteriology 200: e00017-00018). Briefly, bacterial BPL1 cells were recovered from overnight grown cultures, by centrifugation at 12,000xg 15 minutes and washed twice with sodium citrate 50 mM pH 4.7. The bacterial pellet was suspended in sodium citrate 50 mM pH 4.7 and disrupted in a PANDA PLUS 2000 homogenizer (GEA) at 1,000 bar. Insoluble cellular material pellet was collected by centrifugation at 12,000xg 90 minutes, suspended in sodium citrate 50 mM pH 4.7 and extracted for 45 minutes 37°ºC with an equal volume of 1-butanol. The aqueous phase containing LTA was retrieved, freeze-dried, and dissolved in sodium citrate 50 mM pH 4.7 and loaded onto a hydrophobic interaction chromatography column (HiTrap Octyl FF).

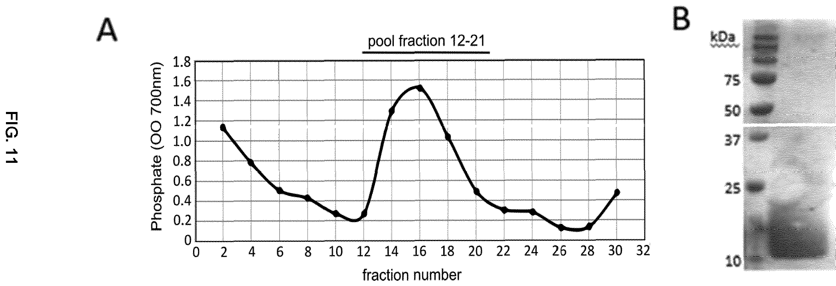

LTA was purified with a linear 15%-65% 1-propanol gradient in sodium citrate 50 mM pH 4.7. Fractions containing LTA were determined by a phosphate assay as described elsewhere (Draing, C., et al. 2006, J Biol Chem 281: 455 33849-33859). Phosphate positive samples were pooled and dialyzed (Figure 11A).

Purified LTA was analyzed by GC-Q-MS, after acid hydrolysis. Briefly, LTA was dissolved at 1 g/L in HCl 2M, and acid hydrolysis conducted at 100ºC for 2 hours. D-glucose, D-galactose, glycerol and glycerol-3-phosphate were determined as their trimethylsilyl derivatives (Fiehn, O., et al. 2000, Anal Chem 72: 3573-3580). GC was conducted in an Agilent 7820A gas chromatographer using a GC column DB5-MS, coupled to a 5977B mass detector, and identification by comparison with Agilent Fiehn GC/MS Metabolomics RTL Library.

### Biochemical characterization of LTAs.

LTA molecules were characterized by matrix-assisted laser desorption ionization-time of flight (MALDI-TOF) mass spectrometry in the proteomics facility of SCSIE University of Valencia (this proteomics laboratory is a member of Proteored PRB3 and is supported by grant PT17/0019 of the PE I+D+I 2013-2016, funded by ISCIII and ERDF). Briefly, 1µl of a sample and 1µl of a matrix solution (10 mg/mL CHCA in 70% ACN, 0.1% TFA) were spotted onto the sample plate. After drying, the sample was analyzed with a mass spetrometer (5800 MALDI TOFTOF, ABSciex) in reflector mode, a mass range of 1,660-1,500 m/z, with lase intensity of 6,000.

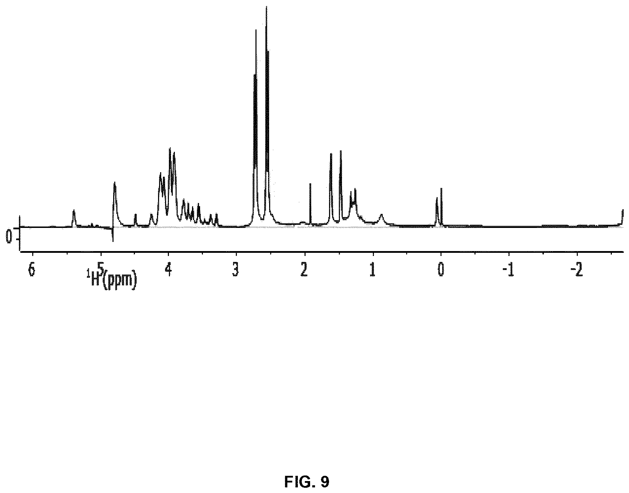

LTA was further analyzed by sodium dodecyl sulfate-polyacrylamide gel electrophoresis (SDS-PAGE). The molecular weight of LTA was shown in the SDS-PAGE gel imaged using the cationic dye Alcian blue coupled with modified silver staining for enhanced sensitivity (Kho and Meredith, 2018, cited *ad supra*). The structure of the LTA was assayed by 1H nuclear magnetic resonance (NMR) spectrometry. Briefly, 1H-NMR was performed on a Bruker AVANCE III 700 Ultrasield spectrometer (Bruekr BioSpin, Rheinstetten, Germany) operating at a 1H frequency of 700.13 MHz, and equipped with a 5mm TCI (cryoprobe) with Z-gradient. The acquisition pulse sequence used were those from Bruker Topspin 3.6 with water presaturation and 2 s recycle time. Spectra was referenced using the TSP signal at 0 ppm (Figure 9).

The purity of the LTA was determined by measuring its endotoxin content with the Limulus amebocyte lysate assay (Lonza Bioscience, Switzerland) since the assay is insensitive to LTA (Morath, S., et al. 2001, J Exp Med 193: 393-397). DNA and RNA contaminations were determined by measuring ultraviolet (UV) absorption at 260 nm and 280 nm (NanoDrop Spectrophotometer, Thermo Fisher Scientific, USA). Sugar component of PG backbone N-acetylmuramic acid was determined by liquid chromatography analysis carried out in an Alliance 2695 HPLC System (Waters Corporation, MA, USA)) coupled to a refractive index detector (model 2414, Waters Corporation MA, USA) and an ion-exchange column (Aminex HPX-87H, Bio-Rad, CA, USA). Aminoacids ornithine, lysine and serine were determined by GC-Q-MS, after acid hydrolysis of purified LTA (HCl 2M, 100ºC, 2 hours) and trimethylsilyl derivatization (Fiehn, O. et al., 2000, cited *ad supra*). GC was conducted in a gas chromatographer (model 7820A, Agilent, CA, USA) using a GC column DB5-MS, coupled to a 5977B mass detector, and identification by comparison with Agilent Fiehn GC/MS Metabolomics RTL Library.

For enzymatic hydrolysis of peptidoglycan, sample of purified LTA was treated (0.04 mg/mL 37ºC, 4 hours) with mutanolysin (SIGMA). After filtration (Amicon Ultra-0.5, Merck Millipore, Germany) soluble muropeptides were reduced by using sodium borohydride at a final concentration of 5 mg/mL. The reaction was stopped after 20 minutes by lowering the pH to 2-4 with phosphoric acid. Fragments were separated with HPLC and a reversed phase octadecyl silica (ODS) C18 column from Phenomenex (CA, USA) Elution was conducted at 30ºC as follows: linear gradient of 0 to 100% buffer B (50mM sodium phosphate pH5.10 with 15% (v/v) methanol) over a period of 120 minutes after 10 minutes in buffer A (50mM sodium phosphate pH4.33) with a flow rate of 0.5 mL per minute. Eluted compounds were detected by monitoring Abs 206 nm (Schaub and Dillard, 2017, cited *ad supra*).

### Statistical analysis

Results are given as the mean ± standard deviation. Data on fat deposition in *C*. *elegans* were analyzed by One Way Anova test, using a Tukey's multiple comparison post-test. To compare effects among different *C*. *elegans* strains, Two-Way Anova test was used. Differences between groups in cell adhesion assays were analyzed by Student's t test. All the statistical analyses were performed with GraphPad Prism 4 software, setting the level of statistical significance at 5%.

### II - RESULTS

To identify the molecule(s) of the BPL1 probiotic strain responsible for the fat reducing functional activity, we used the nematode *C. elegans* as a simple and rapid model to evaluate the fat reduction produced by different cellular fractions. This nematode stores lipids in hypodermic and intestinal cells, easily detected by staining (Nile red). Many proteins involved in lipid synthesis, degradation and transport are conserved between *C. elegans* and mammals.

*A priori* the fat reducing activity might be present in bacterial cells and/or in the culture supernatant. Thus, we tested firstly the culture medium, i.e., age-synchronized nematodes of the wild-type strain N2 were reared and fed with the supernatant obtained after overnight culture of the probiotic BPL1 strain in MRS+Cys medium. No effect on fat reduction was observed in these nematodes when subjected to Nile red staining and subsequent fluorescence quantification in a spectrofluorometer (data not shown). Thus, we can discard that the activity was due to a secretable or released substance during the growth.

Taking into account that one of the main components of the cell is DNA and knowing that DNA from probiotic bacteria has been shown to exert some functional activities, we assayed in *C. elegans,* DNA isolated from BPL1. However, DNA did not display a fat reducing effect (data not shown).

To assess whether soluble or insoluble components of BPL1 cells were responsible of its fat reducing activity, we mechanically disrupted BPL1 cells obtained from an overnight culture and generated insoluble and soluble cellular fractions that were separated by centrifugation. Fluorescence assays revealed fat reduction activity was mainly present in the insoluble fraction (data not shown). This result suggests that the compound could more probably present in the cell envelope as part of the cell wall, membrane surface associated proteins or surface associated polysaccharides, among others.

To test if some proteins associated to cell surface of BPL1 could be responsible of the fat reducing effect, whole cells of BPL1 probiotic strain were treated with proteinase K (50 µg/mL), and then used as feed for nematodes. This treatment does not affect the functional activity of the cells, suggesting that the molecule responsible for the fat reducing effect is not a surface protein or at least it is not degradable by this protease (Figure 1A).

Having gained evidence of the functional activity of BPL1 cell envelope, we designed different strategies to investigate the functionality of the strain when cultured in different media that could modify the composition of the cell envelope. Ampicillin interferes cell wall synthesis by inhibiting the transpeptidase. Vancomycin is a glycopeptide that inhibits cell wall synthesis by blocking the transglycosilation of N-acetylmuramic acid and N-acetylglucosamine. Thus, we cultured BPL1 in the presence of sub-lethal doses of ampicillin or vancomycin and cells were evaluated for functionality. In these conditions, cells can grow but it is expected to alter the properties of the cell envelope. Interestingly, cells obtained under these culture conditions did not exert fat-reducing effects in the nematodes (Figure 1B). This result suggests that an alteration of the cell envelope affects dramatically the fat-reducing properties of the probiotic, reinforcing the hypothesis that the compound is a component of cell envelope.

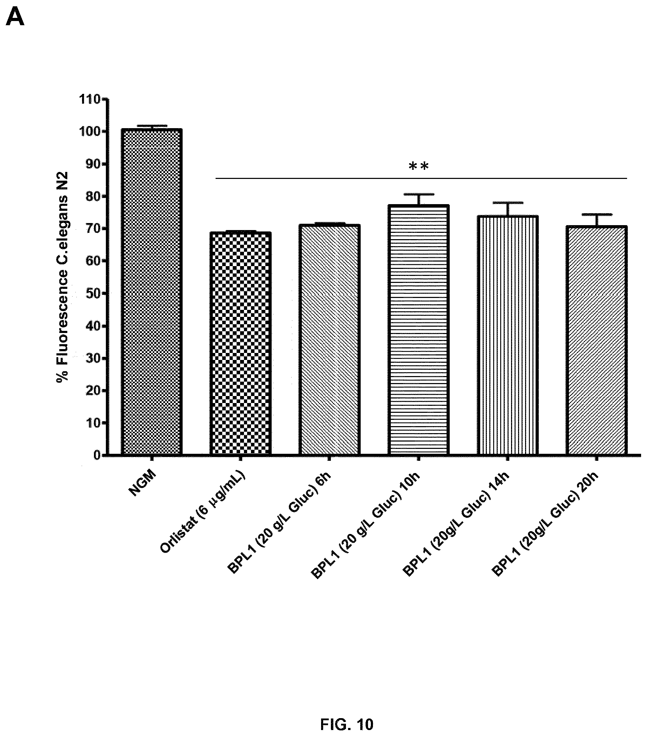

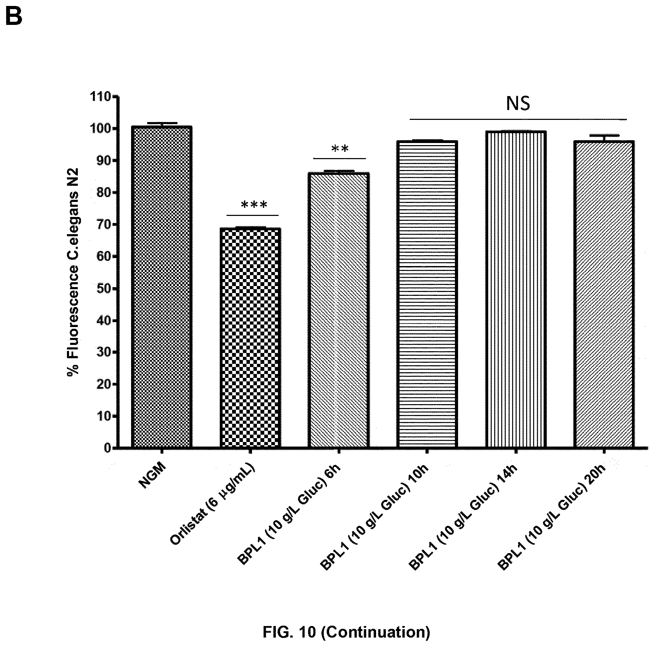

A second strategy to alter the properties of cell envelope was to use glucose restriction conditions in the culture media since glucose is a precursor in the biosynthesis of many envelope components. Cells were overnight cultured in MRS+Cys medium and formulated with different concentrations of glucose. Remarkably, BPL1 cells cultured under glucose restriction lost their fat-reducing capacity in *C. elegans* (Figure 1C). The functional activity of the cells clearly differed depending on glucose availability (Figure 1D). Interestingly, BPL1 cells grown with 20 g/L of glucose (excess of glucose) exerted fat reducing activity when collected in all growth phases but BPL1 cells cultured with restriction of glucose (10 g/L), only exerted a fat reducing effect in the initial exponential phase, when glucose was mainly available (Figure 10A y 10B). Such observation highlights that, in some cases, probiotic functional activity may require particular environmental conditions.

Furthermore, D-glucose was replaced in MRS-Cys medium by different sugars, as fructose, saccharose, lactose, maltose or galactose. Results indicate a loss of fat reducing phenotype of cells grown under sugar restriction in all cases (10g/L), while being functional with high amount of sugar (20g/L) (Figure 1E). The result suggests that the composition of cell envelope is dramatically dependent of the presence of large amounts of sugars used as substrate.

Taking into account that cell adherence could be an important property to exert the fat-reducing activity we investigated if glucose restriction might alter the ability of BPL1 cells to adhere to epithelial cells of the intestinal tract. In order to evaluate this parameter, we used a conventional assay employed to characterize probiotic adhesion potential (Darfeuille-Michaud, A. et al. 1990. Infect Immun 58, 893-902). BPL1 grown in standard MRS-Cys medium (with 20 g/L glucose) exhibited significantly greater adhesion (P<0.01%) to Caco-2 cells compared to the strain grown with 10 g/L glucose (Figure 1D). Therefore, these results reinforce again the idea that the composition of cell envelope from BPL1 is modulated by glucose and this modulation induces large differences in cellular adhesion capacity.

At this stage we investigated which component of cell envelope could be responsible of the observed properties. In this sense, we analyzed the BPL1 cell wall fraction containing peptidoglycan (PG).

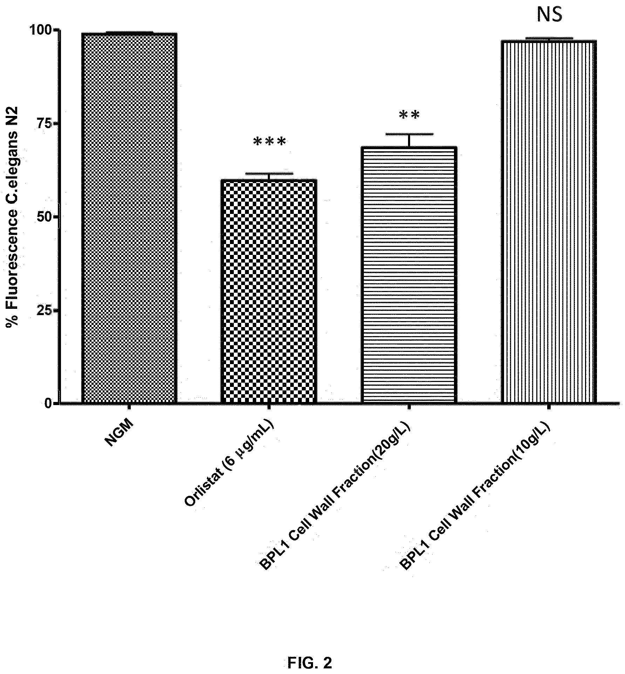

To assess the potential activity of BPL1-derived PG on *C. elegans* fat reduction, we prepared a cell-wall fraction from BPL1 cultured in excess of glucose (MRS-Cys medium) and under restricted glucose concentration. Our results showed that fat-reducing activity was absent when cell wall fraction was prepared from BPL1 cells grown under glucose restriction, while being functional when cells were grown under glucose conditions (Figure 2). This result points out that cell wall fraction or a component extracted together with this fraction is responsible of fat reduction.

To analyze differences within the cell wall fractions obtained with or without glucose restriction we determined the aminoacidic composition of cell wall fraction. However, enantiomeric analysis of amino acids revealed only minor differences between both fractions (data not shown) suggesting that most probably the peptide composition of PG is not responsible for such differences.

Conversely, when we treated cell wall fraction with a muramidase that should destroy its structure we did not observe any decrease in the fat-reducing activity (data not shown) suggesting that probably a component co-purified with the cell wall fraction, but not PG, could be responsible of the activity.

We then focused our interest on lipoteichoic acid (LTA) that sometimes is found as an impurity in cell wall fractions. LTA is an important cell envelope component that is anchored to cell membranes by lipidic moieties. LTA was obtained from the BPL1 cells grown with and without glucose restriction and assessed whether glucose restriction in the BPL1 culture medium affected LTA functionality. LTA fractions were obtained from BPL1 cultures in MRS-Cys with 10 g/L and 20 g/L of glucose and evaluated for their ability to reduce fat in *C. elegans.* LTA extracted from BPL1 cells grown in glucose medium was active, whereas LTA isolated from BPL1 grown in restricted glucose medium was non-functional (Figure 3A and Table 1).

**Table 1: Percentage of fat reduction in C. elegans provided by LTA obtained from BPL 1 and BPL 1 cultured with lower doses of glucose (10 g/L). HT-BPL 1 - Heat- B. lactis CECT 8145.**

| Conditions | % Fat Reduction |
|---|---|
| BPL1 (20g/L glucose) | 31.19 |
| HT-BPL1 | 26.51 |
| BPL1 LTA (20g/L glucose) | 25.60 |
| BPL1 LTA (10g/L glucose) | 2.26 |

Furthermore, the fat reducing activity of LTA fraction was assessed after heat treatment and lyophilization, both preserving its activity (Figure 3B). Finally, the fat-reducing effect of LTA was demonstrated after purification step (Figure 3C). Higher doses of LTA were also tested (20 and 50 µg/mL), but similar reducing effect than 10 µg/mL was observed (data not shown). Lipid storage in nematodes fed with BPL1 cells, heat treated cells (HT-BPL1) and LTA from BPL1 is shown in Figure 3D. As triglycerides (TGs) are the main constituents in lipid droplets stored in *C. elegans,* and lipid accumulation has been associated with increase in TG content in this nematode (Zhang, J., et al. 2011, J Mol Biol 411: 537-553), we further quantified the TGs levels in nematodes fed with the three LTA effective doses. Results indicated a significant reduction in total TG content in animals fed with the LTA (10 µg/mL; P<0.01; 1 and 0.1 µg/mL; P<0.05) (Figure 3E). These results support the total fat reduction observed, and is are consistent with the fat-reducing effect of the probiotic strain BPL1 (and its heat-treated form).

Furthermore, LTA was purified from BPL1 cells cultured in MRS-Cys in glucose limiting conditions, vs standard MRS-Cys medium and evaluated for their ability to reduce fat in *C. elegans.* LTA extracted from BPL1 cells grown in standard glucose medium was active, whereas LTA isolated from BPL1 grown in restricted glucose medium was non-functional (Figure 3F).

Having demonstrated the efficacy of LTA from the BPL1 strain in fat reduction, next we investigated the mechanisms underlying this functional effect. We have previously reported that the fat-reducing and antioxidant activities of BPL1 cells are dependent on the IIS pathway (Martorell, P. et al. 2016. J Agric Food Chem 64, 3462-3472). Due to the evolutionary conservation of the IIS pathway, study of compounds targeting this pathway in *C. elegans* is likely to shed light on its function in higher organisms and humans. To investigate the role of the IIS pathway in LTA-mediated fat reduction, DAF-2 (insulin receptor)/DAF-16, the key regulators in the IIS pathway were evaluated. The fluorescence assays showed that the DAF-16 mutation (*daf-16* (mgDf50)) abolished the LTA-mediated fat-reducing effect (Figure 4A). This was also the case for the BPL1 and HT-BPL1 cells. This result strongly suggests that fat reduction induced by BPL1 is DAF-16 dependent. DAF-2, is the human insulin receptor homolog upstream DAF-16 in the IIS pathway, and as in humans, mutations in the insulin receptor increase fat accumulation, showing an obese phenotype. Our results show that feeding DAF-2 mutant (*daf-2* (e1370)) worms with LTA did not produce a fat-reducing phenotype, indicating that the LTA-mediated regulation of DAF-16 is dependent on DAF-2, and therefore, LTA effect requires the insulin/IGF-I-like signaling pathway (IIS) (Figure 4A). Here, we observed a different response both with BPL1 and HT-BPL1 that is independent of SKN-1, as fat-reducing effects were still observed in a *C*. *elegans* mutant deficient in the ortholog of mammalian Nrf transcription factor (Figure 4B). Similarly, LTA also reduce fat content in *C. elegans* mutant of SKN-1 transcription factor (Figure 4B), suggesting that do not require SKN-1 for its function.

The insulin signaling pathway is also involved in glucose transport, playing a role in glucose homeostasis and insulin sensitivity. Therefore, it is a target pathway for the study of diabetes (or obesity-related diabetes) and the compounds targeting this pathway emerge as potential therapeutics. In *C. elegans,* glucose has been shown to shorten lifespan by up-regulating IGF-I pathway (IIS) activity or increasing reactive oxygen species (ROS) and because of that, it has been suggested that *C*. *elegans* is a good model system to evaluate glucose toxicity and to develop more efficient diabetes therapies.

Thus, we have used high glucose-fed nematodes to model diabetes and evaluate the efficacy of LTA. Taking into account that glucose is a known precursor of triglycerides (TGs) and TGs are the main components of lipid droplets in the nematode, we evaluated the effect of glucose (100 mM) on nematode fat content. Our results showed that fat content increased by 20%, in nematodes fed on high-glucose NGM, in agreement with other reports (Figure 4C). Metformin (biguanide), a drug used in the management of type-2 diabetes, was included as positive control. Furthermore, we evaluated whether LTA could reduce fat content in a diabetic obese *C*. *elegans* model. Hyperglycemic nematodes fed with LTA showed a significant reduction in body fat content (Figure 4C). This effect was also recorded in nematodes fed with BPL1 and HT-BPL1 cells (Figure 4C). These results show the potential of BPL1 cells and its heat-treated form, HT-BPL1, as ingredients for therapeutic and/or preventive uses in diabetes-related obesity. Furthermore, and for the first time, the efficacy of LTA is shown in an *in vivo* hyperglycemic model, highlighting its relevance not only as a functional signaling molecule but also as a postbiotic with proven beneficial effects.

Overall, our findings reveal a previously unrecognized role for LTA as a new lipid modulator exhibiting fat-reducing properties in the pre-clinical model of *C. elegans.*

Our study is not only the first one to show a novel beneficial biological role of LTA from genus *Bifidobacterium,* but also the first one that demonstrates the involvement of LTA in fat-reducing activity using *C. elegans* as a host model. Our results illustrate the potential of LTA obtained from BPL1 probiotic strain as a new postbiotic, having therapeutic and/or preventive application in metabolic syndrome and diabetes-related disorders. Such applications include uses as an ingredient in human nutrition, including food and beverages, nutritional supplements and also medical foods.

### Example 2: LTA from BPL1 has fat reducing activity and the different extraction methods and treatments applied preserve this capacity.

### I - MATERIAL AND METHODS

LTA was obtained from fresh cultures of BPL1 and heat-treated cultures of BPL1, following procedures here described. First, two different cell disruption methods, sonication and PANDA Homogenizer, were compared. Nematodes were cultured in the NGM plates (control conditions) and the NGM plates supplemented with the corresponding LTA from BPL1. Fat content was measured in the different nematode's populations by Nile Red staining method, a fluorescence dye which specifically binds to lipid droplets (Martorell P, et al., 2012, J Agric Food Chem. 60:11071-9).

### II - RESULTS

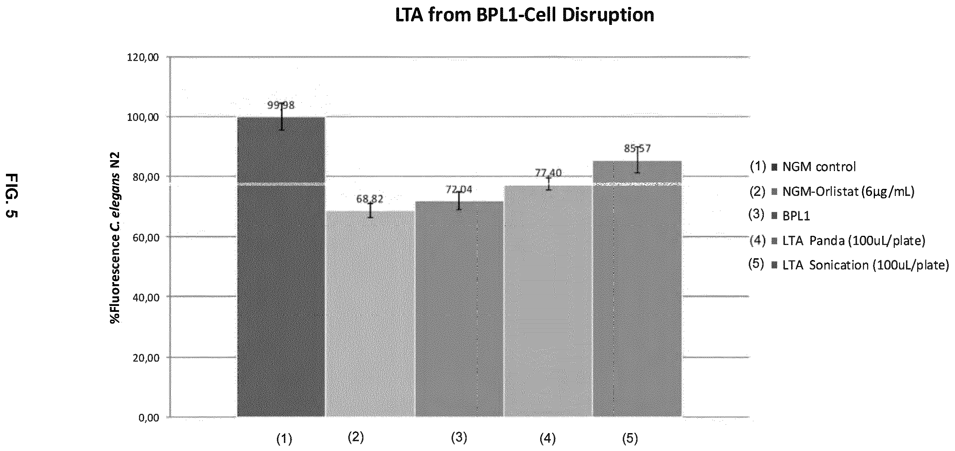

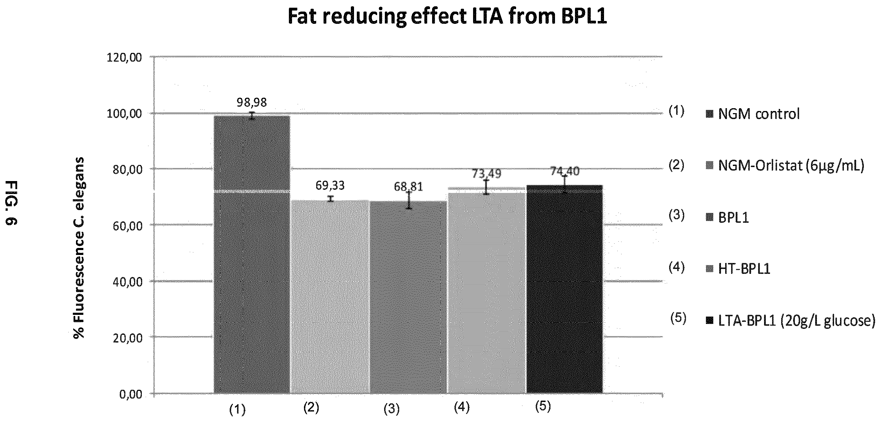

As can be seen from Figure 5 and 6, LTA from BPL1 cultured in excess of glucose has fat-reducing activity and the mechanical disruption provides slightly more effective LTA.

### Example 3: LTA extracted from Bifidobacteria, and LTAs extracted from Lactobacilli and Bacilli strains are shown to exhibit fat-reducing capacity. Bifidobacterial LTAs exhibit higher fat-reducing capacity.

### I - MATERIAL AND METHODS

### Isolation of LTA

*Bifidobacterium* and *Lactobacillus* strains were cultured in MRS (10g/L meat extract, 5g/L yeast extract, 20 g/L D-glucose, 2g/L K2HPO4, 2g/L Di-ammonium hydrogen citrate, 5g/L Sodium Acetate, 0.2g/L MgSO4, 0.05g/L MnSO4, 1 g/L Tween 80) supplemented with 0.05% cysteine). The commercial medium Brain heart infusion CM1135 B (Oxoid) was used to grow *Bacillus* strains. Cultures were overnight incubated under anaerobic conditions at 37ºC (except *Bacillus* strains, incubated in aerobic conditions). Cultures were adjusted to 1x10⁹ cells/mL and washed three times with saline solution. LTA extractions were adapted from Colagiorgi *et al.* 2015 (Colagiorgi A, et al., 2015. FEMS Microbiol Lett. 362: fnv141). Cells were harvested and mechanically disrupted in a PANDA Homogenizer. Afterwards, bacterial lysates were mixed with an equal volume of n-butanol and stirred from 30 minutes at room temperature. Phases were separated by centrifugation for 20 minutes at 13000 x g at 4ºC. The aqueous phase was recovered and subjected to freeze-drying or a further purification step.

For purification, lyophilized samples were suspended with chromatography start buffer (15% n-propanol in 0.1 M ammonium acetate, pH 4.7), and centrifuged at 10,000 rpm for 60 minutes and sterilized by membrane filtration (0.2 µm). The supernatant was subjected to hydrophobic interaction chromatography (HIC) on octyl-Sepharose column (GE Healthcare Life Sciences, UK). Elution was conducted by linear gradient from start buffer to elution buffer (60% n-propanol in 0.1 M ammonium acetate, pH 4.7). The obtained fractions were assessed by a molybdenum blue test to detect phosphate-containing fractions, which were pooled and lyophilized (Villéger R, et al. 2014. Antonie Van Leeuwenhoek. 106:693-706).

### II - RESULTS

LTA was obtained from *B. animalis* subsp. *lactis* BPL1 as described above and compared with other strains belonging to *Bifidobacterium, Lactobacillus* and *Bacillus* genera.

LTAs obtained were added to the nematode culture (NGM). Worms were fed in the different conditions and total fat content was measured by Nile Red staining, a fluorescence dye which specifically binds to lipid droplets, following the same protocol described in example 1.

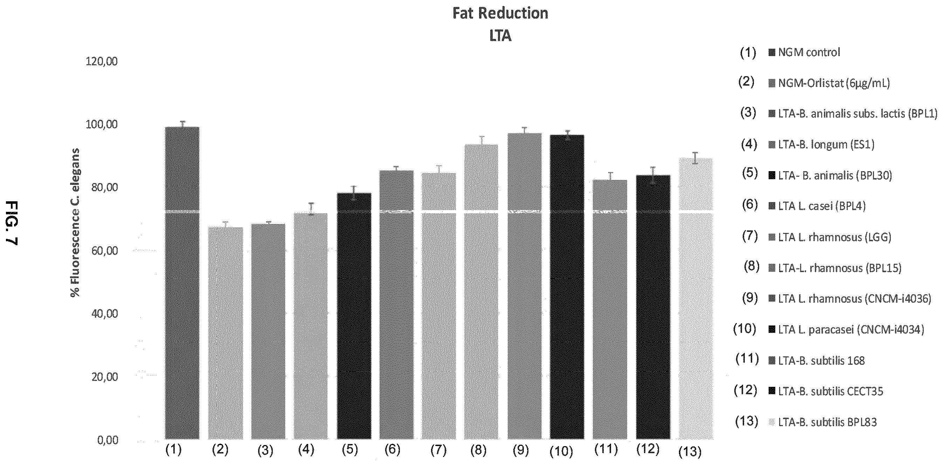

Results indicated that, among *Bifidobacterium* strains, LTA from *B. animalis* subsp. *lactis* BPL1 was the most effective (30.79% of fat reduction), clearly showing the superior activity of BPL1 LTA versus other Bifidobacteria. Moreover, the LTA obtained from *Bifidobacterium* strains exhibited higher fat-reducing effect compared with the LTA from lactobacilli and bacilli strains (Figure 7 and Table 2).

**Table 2: Percentage of fat reduction in C. elegans provided by LTA obtained from BPL1 and other Bifidobacterium, Lactobacillus and Bacillus strains.**

| Conditions | % Fat Reduction |
|---|---|
| LTA- *Bifidobacterium animalis* subsp. *lactis* (CECT8145 or BPL1) | 30.79 |
| LTA- *Bifidobacterium animalis* (BPL30) | 21.05 |
| LTA- *Bifidobacterium longum* (CECT 7347 or ES1) | 26.71 |
| LTA- *Lactobacillus casei* (BPL4) | 13.75 |
| LTA- *Lactobacillus rhamnosus* (LGG) | 14.56 |
| LTA- *L. rhamnosus* (BPL15) | 6,51 |
| LTA- *L. rhamnosus* (CNCM-i4036) | 2,85 |
| LTA- *L. rhamnosus* (CNCM-i4034) | 3,55 |
| LTA- *Bacillus subtilis* 168 | 16.65 |
| LTA- *B. subtilis* CECT35 | 15.29 |
| LTA- *B..subtilis* BPL83 | 9.79 |

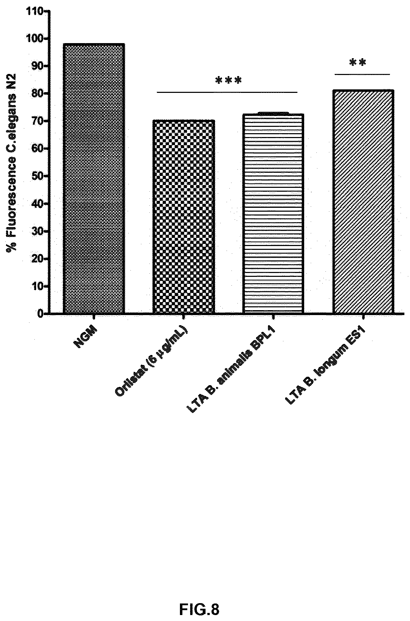

LTAs from other bifidobacterial strains were isolated and purified following the previously described protocols. LTA from *B. longum* ES1 (CECT7347) was functionally evaluated for its fat-reducing capacity in *C. elegans.* Results showed that ES1-LTA significantly reduced nematode's fat content, but in a lesser extent than BPL1-LTA (Figure 8).

### Example 4: Fat-reducing LTA (from BPL1) shows specific structural features when cultured in excess of sugars as carbon source.

### I - MATERIAL AND METHODS

### Isolation of the LTA from Bifidobacteria culture in excess of glucose

The LTA from BPL1 was obtained as described in the above Examples.

### Analysis of the LTA

The structure and composition of LTA obtained from BPL1 has been determined in accordance to the methods described in the literature and shown to contain D-glucose, D-galactose, glycerol, phosphorous and alanine.

Briefly, 1H nuclear magnetic resonance (NMR) experiments were performed on a Bruker AVANCE III 700 Ultrasield spectrometer (Bruekr BioSpin, Rheinstetten, Germany) operating at a 1H frequency of 700.13 MHz, and equipped with a 5mm TCI (cryoprobe) with Z-gradient. The acquisition pulse sequence used were those from Bruker Topspin 3.6 with water presaturation and 2 s recycle time. Spectra were referenced using the TSP signal at 0 ppm.

D-glucose, D-galactose, glycerol, glycerol-P and total alanine were determined as their trimethylsilyl derivatives after acid hydrolysis of samples by gas chromatography performed with an Agilent 7820A gas chromatography system coupled to a 5977B mass detector using a DB5-MS column. The identification was carried out by comparison the retention time and spectral mass with those included in the Agilent Fiehn GC/MS Metabolomics RTL Library.

L- and D- isomers of alanine were determined liquid chromatography analysis carried out in an Alliance 2695 HPLC system, from Waters, coupled to a photodiode array detector (model 2996, Waters). The separation takes place in the chiral column, Chirex 3126 (D)-penicillamine, from Phenomenex, and the identification is by retention time and absorption spectrum, compared to D- and L-Alanine analytical standards

### II - RESULTS

The amount of the different chemical constituents of BPL1 LTA was determined by the techniques above described as is known to the skilled in the state of the art. Briefly, the amount of galactose, glycerol, alanine, glycerol-P, and phosphorous was determined in purified LTA samples, coming from BPL1 cultures grown in the presence of excess glucose (BPL1, 20 g/L glucose) and in limitation of glucose (BPL1, 10 g/L glucose).

As shown in Table 3, the relative content of glycerol, and glycerol-phosphate, vs glucose, and alanine, vs glucose, was found to be surprisingly higher in the LTA sample purified from BPL1 grown in excess of glucose, and exerting fat reduction in C. elegans.

**Table 3. Compositional analysis of BPL1 LTA in different growth conditions.**

| **Source of LTA** | **Molar ratio to glucose** | | | | | **%Fat reduction** |
|---|---|---|---|---|---|---|
| | Galactose | Glycerol | Alanine | Glycerol-P | Phosphorous | |
| *B. animalis* BPL-1 (20g/L glucose) | 0.8 | 2.2 | 1.0 | 12.6 | 26.5 | 26.9 |
| *B. animalis* BPL-1 (10g/L glucose) | 0.6 | 0.7 | 0.2 | 1.1 | 2.6 | 2.0 |

**Table 4. Alanine isomer distribution**

| **Source of LTA** | % **of total Alanine** | |
|---|---|---|
| | L-Alanine | D-Alanine |
| *B. animalis* BPL-1 (20g/L glucose) | 32.4 | 67.6 |
| *B. animalis* BPL-1 (10g/L glucose) | 100 | < LOD |

Results are shown in Figure 8.

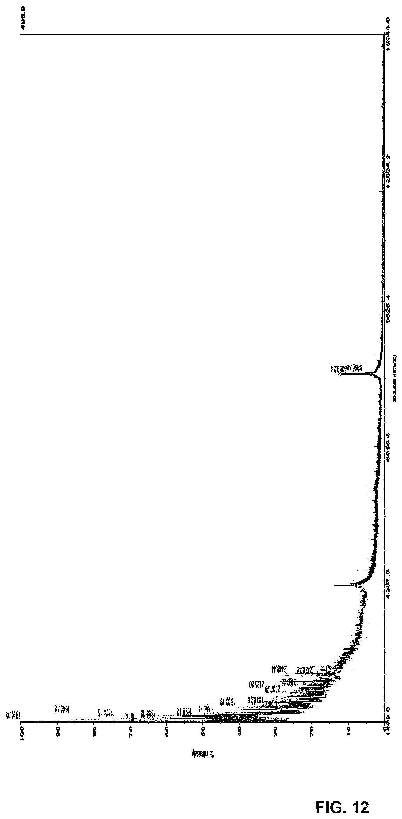

To test this, we isolated and purified LTA from BPL1 strain overnight cultures grown in MRS-Cys .Isolation and purification of LTA was conducted by butanol extraction of the insoluble fraction of BPL1 cellular lysates, followed by hydrophobic exchange chromatography, as previously described to obtain high quality LTA (Morath, et al., 2001 (cited *ad supra*); Draing, et al. 2006 (cited *ad supra*); Gründling and Schneewind, 2007, Proceedings of the National Academy of Sciences 104: 8478-8483; Kho and Meredith, 2018 (cited *ad supra*))*.* A single elution peak was obtained by phosphate assay (Figure 11A). The fractions forming the peak were pooled and used as purified LTA. The purity of LTA with respect to potential cross-contaminants, cell-wall material and nucleic acid was determined by specific analysis. Endotoxin contamination was excluded through an LAL assay and endotoxin content was <5 EU/mg in the lyophilized LTA. Nucleic acids contamination was determined by measuring UV absorption at 260 nm and 280 nm. DNA/RNA accounted for less than 1.5% wt/wt. Peptidoglycan contamination was excluded by analysis of derived muropeptides after mutanolysin treatment in which no peaks of peptidoglycan fragments were detected. In addition, N-acetylmuramic acid (MurNAc) or the aminoacids ornithine, lysine and serine, known to be present in the PG of BPL1 (previously determined by 1D- and 2D-TLC of the total hydrolysate of the peptidoglycan of BPL1, data not shown), were not detected by gas or liquid chromatography of the total hydrolysate of purified LTA. To further characterize purified LTA, a sample was stained through Alcian blue/silver staining following SDS-PAGE (Figure 11B), and analyzed using MALDI-TOF mass spectrometry and nuclear magnetic resonance (Figure 12), revealing a compound with an estimated molecular mass distribution in the range of 8-10 kDa.

## Claims

1. A lipoteichoic acid (LTA) comprising a molar ratio Alanine/glucose of at least 0.5 and a molar ratio glycerol phosphate/glucose of at least 6.0, preferably wherein said LTA is heat-treated or lyophilized.

2. LTA according to claim 1, wherein the molar ratio Alanine/glucose is at least 0.6, 0.7, 0.8, 0.9 or 1.0.

3. LTA according to claim 1 or 2, wherein the Alanines of the LTA are L-Alanine, D-Alanine or a combination of L- and D- Alanines.

4. LTA according to any one of claims 1 to 3, wherein the molar ratio glycerol phosphate/glucose is at least 6.5, 7.0, 7.5, 8.0, 8.5, 9.0, 9.5, 10.0, 10.5, 11.0, 11.5, 12.0 or 12.5, preferably 12.6.

5. A composition comprising the LTA according to any one of claims 1 to 4.

6. Composition according to claim 5, further comprising a bioactive compound.

7. Composition according to claim 5 or 6, wherein the composition is a pharmaceutical composition or a nutritional composition.

8. A LTA according to any one of claims 1 to 4, or a composition according to any one of claims 5 to 7, for use as a medicament, preferably, for use in the treatment and/or prevention of obesity, overweight or a related disease selected from the group consisting of diabetes, metabolic syndrome, hypertension, hyperglycaemia, inflammation, type-2 diabetes, cardiovascular disease, hypercholesterolemia, hormonal disorders and infertility.

9. A non-therapeutic use of a LTA according to any one of claims 1 to 4, or a composition according to any one of claims 5 to 7, for body fat reduction.

10. Use of a LTA according to any one of claims 1 to 4, or a composition according to any one of claims 5 to 7, for the elaboration of a food or feed product.

11. A process for obtaining a LTA from bifidobacteria, wherein said LTA shows the capacity of reducing body fat in a subject, comprising the following steps:
(a) cultivating the strain *Bifidobacterium animalis* subsp. *lactis* CECT 8145 in excess of sugars as carbon source, and
(b) isolating the LTA from the cell wall of the bacterium.

12. A LTA from bifidobacteria cultured in excess of sugars as carbon source for use as a medicament, preferably, for use in the treatment and/or prevention of obesity, overweight or related diseases selected from the group consisting of diabetes, metabolic syndrome, hypertension, hyperglycaemia, inflammation, type-2 diabetes, cardiovascular disease, hypercholesterolemia, hormonal disorders and infertility, wherein the structure of the LTA is the following: 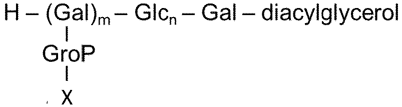 wherein
- GroP is glycerophosphate,
- X is Alanine (Ala) or Hydrogen (H),
- Gal is galactofuranan,
- each X is independently selected from hydrogen and Alanine (Ala),
- the number of molecules of Alanine is m or m-p, being m the number of repeating units of Gal and p the number of units of Gal in which X is hydrogen,
- Glc is glucan,
- m is the number of repeating units of Gal and is between 10 and 20, preferably, between 11 to 18, and
- n is the number of repeating units of Glc and is between 5 and 20, preferably, between 8 and 12,
preferably wherein said LTA is heat-treated or lyophilized.

13. Non-therapeutic use of a LTA from bifidobacteria cultured in excess of sugars as carbon source for body fat reduction, wherein the structure of the LTA is the following: 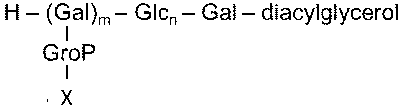 wherein
- GroP is glycerophosphate,
- X is Alanine (Ala) or Hydrogen (H),
- Gal is galactofuranan,
- each X is independently selected from hydrogen and Alanine (Ala),
- the number of molecules of Alanine is m or m-p, being m the number of repeating units of Gal and p the number of units of Gal in which X is hydrogen,
- Glc is glucan,
- m is the number of repeating units of Gal and is between 10 and 20, preferably, between 11 to 18, and
- n is the number of repeating units of Glc and is between 5 and 20, preferably, between 8 and 12,
preferably wherein said LTA is heat-treated or lyophilized.

14. Use of a LTA from bifidobacteria cultured in excess of sugars as carbon source for the elaboration of a food or feed product, preferably a nutritional supplement, wherein the structure of the LTA is the following: 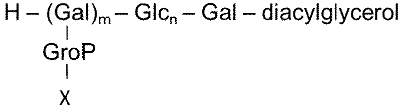 wherein
- GroP is glycerophosphate,
- X is Alanine (Ala) or Hydrogen (H),
- Gal is galactofuranan,
- each X is independently selected from hydrogen and Alanine (Ala),
- the number of molecules of Alanine is m or m-p, being m the number of repeating units of Gal and p the number of units of Gal in which X is hydrogen,
- Glc is glucan,
- m is the number of repeating units of Gal and is between 10 and 20, preferably, between 11 to 18, and
- n is the number of repeating units of Glc and is between 5 and 20, preferably, between 8 and 12,
preferably wherein said LTA is heat-treated or lyophilized.

## Patentansprüche

1. Lipoteichoicsäure (lipoteichoic acid, LTA), umfassend ein Molverhältnis Alanin/Glucose von mindestens 0,5 und ein Molverhältnis Glycerinphosphat/Glucose von mindestens 6,0, wobei die LTA vorzugsweise wärmebehandelt oder lyophilisiert ist.

2. LTA nach Anspruch 1, wobei das Molverhältnis Alanin/Glucose mindestens 0,6, 0,7, 0,8, 0,9 oder 1,0 beträgt.

3. LTA nach Anspruch 1 oder 2, wobei die Alanine der LTA L-Alanin, D-Alanin oder eine Kombination aus L- und D-Alaninen sind.

4. LTA nach einem der Ansprüche 1 bis 3, wobei das Molverhältnis Glycerinphosphat/Glucose mindestens 6,5, 7,0, 7,5, 8,0, 8,5, 9,0, 9,5, 10,0, 10,5, 11,0, 11,5, 12,0 oder 12,5, vorzugsweise 12,6 beträgt.

5. Zusammensetzung, umfassend die LTA nach einem der Ansprüche 1 bis 4.

6. Zusammensetzung nach Anspruch 5, ferner umfassend eine bioaktive Verbindung.

7. Zusammensetzung nach Anspruch 5 oder 6, wobei die Zusammensetzung eine pharmazeutische Zusammensetzung oder eine Nahrungszusammensetzung ist.

8. LTA nach einem der Ansprüche 1 bis 4 oder eine Zusammensetzung nach einem der Ansprüche 5 bis 7 zur Verwendung als Arzneimittel, vorzugsweise zur Verwendung bei der Behandlung und/oder Vorbeugung von Fettleibigkeit, Übergewicht oder einer damit zusammenhängenden Krankheit, ausgewählt aus der Gruppe bestehend aus Diabetes, metabolischem Syndrom, Bluthochdruck, Hyperglykämie, Entzündung, Typ-2-Diabetes, kardiovaskulären Erkrankungen, Hypercholesterinämie, hormonellen Störungen und Unfruchtbarkeit.

9. Nicht-therapeutische Verwendung einer LTA nach einem der Ansprüche 1 bis 4 oder einer Zusammensetzung nach einem der Ansprüche 5 bis 7 zur Körperfettreduktion.

10. Verwendung einer LTA nach einem der Ansprüche 1 bis 4 oder einer Zusammensetzung nach einem der Ansprüche 5 bis 7 zur Herstellung eines Lebens- oder Futtermittels.

11. Verfahren zur Gewinnung einer LTA aus einem Bidifobakterium, wobei die genannte LTA in der Lage ist, das Körperfett einer Person zu reduzieren, umfassend die folgenden Schritte:
(a) Züchten eines *Bifidobacterium animalis* subsp. *lactis* CECT 8145 mit einem Überschuss an Zuckern als Kohlenstoffquelle und
(b) Isolieren der LTA aus der Zellwand des Bakteriums.

12. LTA aus Bifidobakterien, die in einem Überschuss an Zuckern als Kohlenstoffquelle gezüchtet wurden, zur Verwendung als Medikament, vorzugsweise zur Verwendung bei der Behandlung und/oder Vorbeugung von Fettleibigkeit, Übergewicht oder verwandten Krankheiten, ausgewählt aus der Gruppe bestehend aus Diabetes, metabolischem Syndrom, Bluthochdruck, Hyperglykämie, Entzündung, Typ-2-Diabetes, kardiovaskulären Erkrankungen, Hypercholesterinämie, hormonellen Störungen und Unfruchtbarkeit, wobei die Struktur der LTA die folgende ist: 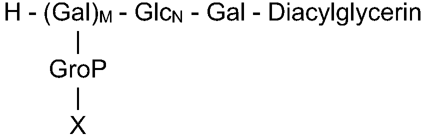 wobei
- GroP Glycerophosphat ist,
- X Alanin (Ala) oder Wasserstoff (H) ist,
- Gal Galactofuranan ist,
- jedes X unabhängig voneinander ausgewählt aus Wasserstoff und Alanin (Ala) ist,
- die Anzahl der Alaninmoleküle m oder m-p ist, wobei m die Anzahl der Wiederholungseinheiten von Gal und p die Anzahl der Gal-Einheiten ist, in denen X Wasserstoff ist,
- Glc Glucan ist,
- m die Anzahl der Wiederholungseinheiten von Gal ist und zwischen 10 und 20, vorzugsweise zwischen 11 und 18 liegt, und
- n die Anzahl der Wiederholungseinheiten von Glc ist und zwischen 5 und 20, vorzugsweise zwischen 8 und 12 liegt,
wobei das LTA vorzugsweise wärmebehandelt oder lyophilisiert ist.

13. Nicht-therapeutische Verwendung einer LTA aus Bifidobakterien, die mit einem Überschuss an Zuckern gezüchtet wurden, als Kohlenstoffquelle zur Reduzierung von Körperfett, wobei die Struktur der LTA die folgende ist: 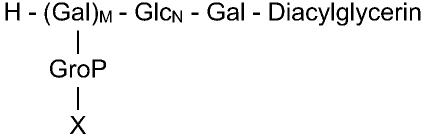 wobei
- GroP Glycerophosphat ist,
- X Alanin (Ala) oder Wasserstoff (H) ist,
- Gal Galactofuranan ist,
- jedes X unabhängig voneinander ausgewählt aus Wasserstoff und Alanin (Ala) ist,
- die Anzahl der Alaninmoleküle m oder m-p ist, wobei m die Anzahl der Wiederholungseinheiten von Gal und p die Anzahl der Gal-Einheiten ist, in denen X Wasserstoff ist,
- Glc Glucan ist,
- m die Anzahl der Wiederholungseinheiten von Gal ist und zwischen 10 und 20, vorzugsweise zwischen 11 und 18 liegt, und
- n die Anzahl der Wiederholungseinheiten von Glc ist und zwischen 5 und 20, vorzugsweise zwischen 8 und 12 liegt,
wobei das LTA vorzugsweise wärmebehandelt oder lyophilisiert ist.

14. Verwendung einer LTA aus Bifidobakterien, die mit einem Überschuss an Zuckern gezüchtet wurden, als Kohlenstoffquelle für die Herstellung eines Lebens- oder Futtermittels, vorzugsweise eines Nahrungsergänzungsmittels, wobei die Struktur der LTA die folgende ist: 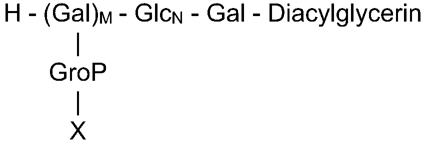 wobei
- GroP Glycerophosphat ist,
- X Alanin (Ala) oder Wasserstoff (H) ist,
- Gal Galactofuranan ist,
- jedes X unabhängig voneinander ausgewählt aus Wasserstoff und Alanin (Ala) ist,
- die Anzahl der Alaninmoleküle ist m oder m-p, wobei m die Anzahl der Wiederholungseinheiten von Gal und p die Anzahl der Gal-Einheiten ist, in denen X Wasserstoff ist,
- Glc Glucan ist,
- m die Anzahl der Wiederholungseinheiten von Gal ist und zwischen 10 und 20, vorzugsweise zwischen 11 und 18 liegt, und
- n die Anzahl der Wiederholungseinheiten von Glc ist und zwischen 5 und 20, vorzugsweise zwischen 8 und 12 liegt,
wobei das LTA vorzugsweise wärmebehandelt oder lyophilisiert ist.

## Revendications

1. Acide lipotéichoïque (LTA) comprenant un rapport molaire alanine/glucose d'au moins 0,5 et un rapport molaire phosphate de glycérol/glucose d'au moins 6,0, de préférence, dans lequel ledit LTA est traité thermiquement ou lyophilisé.

2. LTA selon la revendication 1, dans lequel le rapport molaire alanine/glucose est d'au moins 0,6, 0,7, 0,8, 0,9 ou 1,0.

3. LTA selon la revendication 1 ou 2, dans lequel les alanines du LTA sont la L-alanine, la D-alanine ou une combinaison de L- et D-alanines.

4. LTA selon l'une quelconque des revendications 1 à 3, dans lequel le rapport molaire phosphate de glycérol/glucose est d'au moins 6,5, 7,0, 7,5, 8,0, 8,5, 9,0, 9,5, 10,0, 10,5, 11,0, 11,5, 12,0 ou 12,5, de préférence 12,6.

5. Composition comprenant le LTA selon l'une quelconque des revendications 1 à 4.

6. Composition selon la revendication 5, comprenant en outre un composé bioactif.

7. Composition selon la revendication 5 ou 6, dans laquelle la composition est une composition pharmaceutique ou une composition nutritionnelle.

8. LTA selon l'une quelconque des revendications 1 à 4, ou composition selon l'une quelconque des revendications 5 à 7, pour une utilisation en guise de médicament, de préférence, pour une utilisation dans le traitement et/ou la prévention de l'obésité, du surpoids ou d'une maladie apparentée choisie dans le groupe constitué de diabète, syndrome métabolique, hypertension, hyperglycémie, inflammation, diabète de type 2, maladie cardiovasculaire, hypercholestérolémie, troubles hormonaux et infertilité.

9. Utilisation non thérapeutique d'un LTA selon l'une quelconque des revendications 1 à 4, ou d'une composition selon l'une quelconque des revendications 5 à 7, pour une réduction de graisse corporelle.

10. Utilisation d'un LTA selon l'une quelconque des revendications 1 à 4, ou d'une composition selon l'une quelconque des revendications 5 à 7, pour l'élaboration d'un aliment ou produit d'alimentation.

11. Procédé destiné à l'obtention d'un LTA provenant de bifidobactéries, dans lequel ledit LTA présente la capacité de réduire la graisse corporelle chez un sujet, comprenant les étapes suivantes :
(a) culture de la souche *Bifidobacterium animalis* subsp. *lactis* CECT 8145 en excès de sucres en guise de source de carbone, et
(b) isolement du LTA de la paroi cellulaire de la bactérie.

12. LTA provenant de bifidobactéries cultivées en excès de sucres en guise de source de carbone pour une utilisation en guise de médicament, de préférence, pour une utilisation dans le traitement et/ou la prévention de l'obésité, du surpoids ou de maladies apparentées choisies dans le groupe constitué de diabète, syndrome métabolique, hypertension, hyperglycémie, inflammation, diabète de type 2, maladie cardiovasculaire, hypercholestérolémie, troubles hormonaux et infertilité, dans lequel la structure du LTA est la suivante : 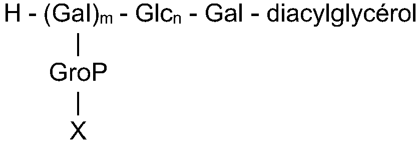 dans lequel
- GroP est un glycérophosphate,
- X est une alanine (Ala) ou un hydrogène (H),
- Gal est un galactofuranane,
- chaque X est choisi indépendamment parmi un hydrogène et une alanine (Ala),
- le nombre de molécules d'alanine est m ou m-p, étant m le nombre de motifs répétés de Gal et p le nombre de motifs de Gal dans lesquels X est un hydrogène,
- Glc est un glucane,
- m est le nombre de motifs répétés de Gal et est compris entre 10 et 20, de préférence, entre 11 et 18, et
- n est le nombre de motifs répétés de Glc et est compris entre 5 et 20, de préférence, entre 8 et 12,
de préférence, dans lequel ledit LTA est traité thermiquement ou lyophilisé.

13. Utilisation non thérapeutique d'un LTA provenant de bifidobactéries cultivées en excès de sucres en guise de source de carbone pour une réduction de graisse corporelle, dans laquelle la structure du LTA est la suivante : 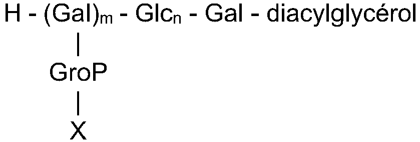 dans lequel
- GroP est un glycérophosphate,
- X est une alanine (Ala) ou un hydrogène (H),
- Gal est un galactofuranane,
- chaque X est choisi indépendamment parmi un hydrogène et une alanine (Ala),
- le nombre de molécules d'alanine est m ou m-p, étant m le nombre de motifs répétés de Gal et p le nombre de motifs de Gal dans lesquels X est un hydrogène,
- Glc est un glucane,
- m est le nombre de motifs répétés de Gal et est compris entre 10 et 20, de préférence, entre 11 et 18, et
- n est le nombre de motifs répétés de Glc et est compris entre 5 et 20, de préférence, entre 8 et 12,
de préférence, dans lequel ledit LTA est traité thermiquement ou lyophilisé.

14. Utilisation d'un LTA provenant de bifidobactéries cultivées en excès de sucres en guise de source de carbone pour l'élaboration d'un aliment ou produit d'alimentation, de préférence un complément nutritionnel, dans laquelle la structure du LTA est la suivante : 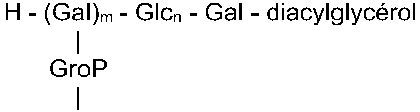 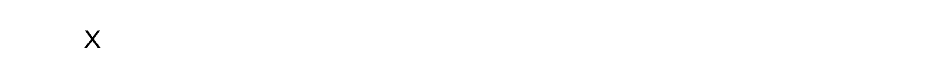 dans lequel
- GroP est un glycérophosphate,
- X est une alanine (Ala) ou un hydrogène (H),
- Gal est un galactofuranane,
- chaque X est choisi indépendamment parmi un hydrogène et une alanine (Ala),
- le nombre de molécules d'alanine est m ou m-p, étant m le nombre de motifs répétés de Gal et p le nombre de motifs de Gal dans lesquels X est un hydrogène,
- Glc est un glucane,
- m est le nombre de motifs répétés de Gal et est compris entre 10 et 20, de préférence, entre 11 et 18, et
- n est le nombre de motifs répétés de Glc et est compris entre 5 et 20, de préférence, entre 8 et 12,
de préférence, dans lequel ledit LTA est traité thermiquement ou lyophilisé.
